(19) Europäisches Patentamt
European Patent Office
Office européen des brevets

(11) Veröffentlichungsnummer: **0 248 414 B1**

# (12) EUROPÄISCHE PATENTSCHRIFT

(45) Veröffentlichungstag der Patentschrift: **23.09.92**

(51) Int. Cl.$^5$: **C07D 233/90**, A61K 31/415

(21) Anmeldenummer: **87108017.2**

(22) Anmeldetag: **03.06.87**

Die Akte enthält technische Angaben, die nach dem Eingang der Anmeldung eingereicht wurden und die nicht in dieser Patentschrift enthalten sind.

## (54) Aralkylimidazolderivate.

(30) Priorität: **06.06.86 CH 2294/86**
**09.10.86 CH 4034/86**

(43) Veröffentlichungstag der Anmeldung:
**09.12.87 Patentblatt 87/50**

(45) Bekanntmachung des Hinweises auf die Patenterteilung:
**23.09.92 Patentblatt 92/39**

(84) Benannte Vertragsstaaten:
**AT BE CH DE ES FR GB GR IT LI LU NL SE**

(56) Entgegenhaltungen:
**EP-A- 0 028 833**
**FR-A- 2 382 443**
**GB-A- 1 044 933**

(73) Patentinhaber: **CIBA-GEIGY AG**
**Klybeckstrasse 141**
**CH-4002 Basel(CH)**

(72) Erfinder: **Allgeier, Hans, Dr.**
**Lichsenweg 20**
**W-7850 Lörrach-Haagen(DE)**

(74) Vertreter: **Zumstein, Fritz, Dr. et al**
**Bräuhausstrasse 4**
**W-8000 München 2(DE)**

**EP 0 248 414 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Aralkylimidazolderivate, d.h. Verbindungen der Formel

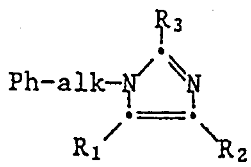

(I),

worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ für gegebenenfalls ein- oder zweifach durch $C_1$-$C_7$-Alkyl substituiertes Carbamoyl und der andere für Wasserstoff, $C_1$-$C_7$-Alkyl oder gegebenenfalls ein- oder zweifach durch $C_1$-$C_7$-Alkyl substituiertes Carbamoyl steht und $R_3$ Wasserstoff oder $C_1$-$C_7$-Alkyl darstellt, mit der Maßgabe, daß in Verbindungen der Formel I, worin Ph-alk Monohalogen-phenyl-$C_1$-$C_2$-alkyl ist, $R_3$ für Wasserstoff steht, wenn einer der Reste $R_1$ und $R_2$ Carbamoyl ist und der andere Wasserstoff ist, zur Anwendung in einem Verfahren zur therapeutischen Behandlung des menschlichen oder tierischen Körpers, diese enthaltende pharmazeutische Präparate und ihre Verwendung als Arzneimittelwirkstoffe sowie die Verbindungen selbst mit der weiteren Maßgabe, daß in Verbindungen der Formel I, in denen $R_1$ und $R_2$ gleich sind und jeweils für Carbamoyl oder N-Methylcarbamoyl stehen, mindestens ein Substituent von Ph in einer o-Stellung gebunden ist, wenn $R_3$ Wasserstoff darstellt, und ein Verfahren zu ihrer Herstellung.

Der Phenylrest Ph enthält bis und mit 3, insbesondere 1 oder 2, der genannten Substituenten, vorzugsweise 1 $C_1$-$C_7$-Alkyl- oder Halogensubstituenten oder 2 Halogensubstituenten, ferner 1 $C_1$-$C_7$-Alkyl- und 1 Halogensubstituenten, wobei vorzugsweise mindestens einer der genannten Substituenten in einer o-Stellung gebunden ist. Als Beispiele seien genannt: o-Halogenphenyl, 2,6-, ferner 2,5-, 2,3- und 2,4-Dihalogenphenyl sowie o-$C_1$-$C_7$-Alkylphenyl, insbesondere 2,6-Difluorphenyl.

Gegebenenfalls ein- oder zweifach durch $C_1$-$C_7$-Alkyl substituiertes Carbamoyl $R_2$ und gegebenenfalls $R_1$ ist beispielsweise Carbamoyl oder N-$C_1$-$C_7$-Alkyl- oder N,N-Di-$C_1$-$C_7$-alkyl-carbamoyl.

Nachstehend sind unter "niederen" organischen Gruppen und Verbindungen solche zu verstehen, die 1 bis und mit 7, insbesondere 1 bis und mit 4, Kohlenstoffatome (C-Atome) enthalten.

$C_1$-$C_7$-Alkyl ist beispielsweise $C_1$-$C_4$-Alkyl, wie Methyl, Ethyl, Propyl, Isopropyl oder Butyl, ferner Sekundärbutyl, Isobutyl oder Tertiärbutyl, kann aber auch eine $C_5$-$C_7$-Alkyl-, wie Pentyl-, Hexyl- oder Heptylgruppe sein.

Halogen ist Halogen mit einer Atomnummer bis und mit 35, d.h. Fluor, Chlor oder in zweiter Linie Brom.

$C_1$-$C_4$-Alkyliden ist insbesondere $C_1$-$C_4$-Alk-1-yliden, wie Methylen, ferner Ethyliden, Prop-1-yliden oder But-1-yliden, kann aber auch $C_3$-$C_4$-Alk-2-yliden, d.h. Prop-2-yliden (Isopropyliden) oder But-2-yliden, sein.

N-$C_1$-$C_7$-Alkylcarbamoyl ist insbesondere N-$C_1$-$C_4$-Alkylcarbamoyl, wie N-Methyl- oder N-Ethylcarbamoyl.

N,N-Di-$C_1$-$C_7$-alkylcarbamoyl ist beispielsweise N,N-Di-$C_1$-$C_4$-alkylcarbamoyl, wie N,N-Dimethylcarbamoyl, ferner N,N-Diethyl- oder N,N-Di-(n-propyl)-carbamoyl.

Die Verbindungen der Formel I besitzen wertvolle pharmakologische Eigenschaften, insbesondere eine ausgeprägte antikonvulsive Wirksamkeit, die sich beispielsweise an der Maus anhand eines deutlichen Metrazol-Antagonismus im Dosisbereich ab etwa 10 mg/kg p.o. sowie an Maus und Ratte anhand einer ausgeprägten Schutzwirkung gegen durch Elektroschock ausgelöste Konvulsionen im Dosisbereich ab etwa 3,5 mg/kg p.o. Zeigen läßt. Die Verbindungen der Formel I sind dementsprechend vorzüglich geeignet zur Behandlung von Konvulsionen verschiedenartiger Genese, beispielsweise zur Behandlung der Epilepsie. Sie können dementsprechend als antikonvulsive, beispielsweise antiepileptische, Arzneimittelwirkstoffe verwendet werden.

In der europäischen patentanmeldung Nr. 28,833 werden unter anderem bereits 2-alkylsubstituierte 1-Monohalogenphenyl-$C_1$-$C_2$-alkylimidazol-(4- oder 5-)carboxamide mit Angiotensin-II-antagonistischer und hypotensiver Wirksamkeit generisch offenbart, welche durch die erste der beiden vorstehend erwähnten Maßgaben aus dem Umfang der vorliegenden Erfindung herausgenommen werden. Weiterhin sind aus FR-A-2,382,443 eine Reihe von spezifisch substituierten 1-Phenylalkylimidazol-4,5-dicarboxamid-Derivaten mit herbizider Wirksamkeit bekannt, welche durch die zweite der beiden vorstehend erwähnten Maßgaben aus dem Umfang der vorliegenden Erfindung herausgenommen werden.

Die Erfindung betrifft in erster Linie Verbindungen der Formel I, worin, unter Berücksichtigung der beiden vorstehend erwähnten Maßgaben, Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ Carbamoyl, N-$C_1$-$C_7$-AlkylcarbamoYl oder N,N-Di-$C_1$-$C_4$-alkylcarbamoyl bedeutet und der andere Wasserstoff, $C_1$-$C_7$-Alkyl, Carbamoyl, N-$C_1$-$C_7$-Alkylcarbamoyl oder N,N-Di-$C_1$-$C_4$-alkylcarbamoyl darstellt und $R_3$ für Wasserstoff oder $C_1$-$C_7$-Alkyl steht, vorzugsweise solche, in denen, unter Berücksichtigung der beiden vorstehend erwähnten Maßgaben, mindestens einer der genannten Substituenten von Ph in o-Stellung gebunden ist und/oder alk Methylen, $R_1$ Wasserstoff oder Carbamoyl und $R_2$ Carbamoyl darstellt.

Die Erfindung betrifft vor allem Verbindungen der Formel I, worin, unter Berücksichtigung der zweiten der vorstehend erwähnten Maßgaben, Ph durch $C_1$-$C_4$-Alkyl monosubstituiertes oder durch Halogen mit einer Atomnummer bis und mit 35 oder Halogen mit einer Atomnummer bis und mit 35 sowie $C_1$-$C_4$-Alkyl disubstituiertes Phenyl bedeutet, wobei $C_1$-$C_4$-Alkyl z.B. für Methyl steht und Halogen z.B. für Fluor oder in zweiter Linie für Chlor steht, alk $C_1$-$C_4$-Alk-1-yliden, wie Methylen oder Ethyliden, oder $C_3$-$C_4$-Alk-2-yliden darstellt, einer der Reste $R_1$ und $R_2$ Carbamoyl oder N-$C_1$-$C_4$-Alkyl- oder N,N-Di-$C_1$-$C_4$-alkylcarbamoyl, wie N-Methyl- oder N,N-Dimethylcarbamoyl, darstellt und der andere Wasserstoff, Carbamoyl oder N-$C_1$-$C_4$-Alkyl- oder N,N-Di-$C_1$-$C_4$-alkylcarbamoyl, wie N-Methyl- oder N,N-Dimethylcarbamoyl, bedeutet und $R_3$ für Wasserstoff oder in zweiter Linie für $C_1$-$C_4$-Alkyl, wie Methyl, steht, vorzugsweise solche, in denen, unter Berücksichtigung der zweiten der vorstehend erwähnten Maßgaben, mindestens einer der genannten Substituenten von Ph in o-Stellung gebunden ist und/oder alk Methylen, $R_1$ Wasserstoff oder Carbamoyl und $R_2$ Carbamoyl darstellt.

Die Erfindung betrifft vorzugsweise Verbindungen der Formel I, worin Ph o-$C_1$-$C_4$-Alkylphenyl, o-Halogenphenyl oder 2,6-, ferner 2,5-Dihalogenphenyl bedeutet, wobei $C_1$-$C_4$-Alkyl z.B. für Methyl steht und Halogen jeweils eine Atomnummer bis und mit 35 aufweist und unabhängig voneinander Fluor oder in zweiter Linie Chlor oder Brom bedeutet, alk $C_1$-$C_4$-Alk-1-yliden, wie Methylen, darstellt, einer der Reste $R_1$ und $R_2$ Carbamoyl, N-$C_1$-$C_4$-Alkylcarbamoyl, wie N-Methylcarbamoyl oder N,N-Di-$C_1$-$C_4$-alkylcarbamoyl, wie N,N-Dimethylcarbamoyl, und der andere Wasserstoff, Carbamoyl, N-$C_1$-$C_4$-Alkylcarbamoyl, wie N-Methyl- oder N-Ethylcarbamoyl, oder N,N-Di-$C_1$-$C_4$-alkylcarbamoyl, wie N,N-Dimethylcarbamoyl, bedeutet und $R_3$ für Wasserstoff steht, vorzugsweise solche, in denen alk Methylen, $R_1$ Wasserstoff oder Carbamoyl und $R_2$ Carbamoyl darstellt und ganz besonders solche, in denen alk Methylen, $R_1$ Wasserstoff und $R_2$ Carbamoyl darstellt.

Die Erfindung betrifft insbesondere einerseits Verbindungen der Formel I, worin Ph o-$C_1$-$C_4$-Alkylphenyl, wie o-Methylphenyl, o-Halogenphenyl, wie o-Chlor- oder o-Fluorphenyl, oder 2,6-Dihalogenphenyl, wie 2,6-Difluorphenyl, bedeutet, wobei Halogen jeweils eine Atomnummer bis und mit 35 aufweisen kann, alk für Methylen steht, $R_1$ Wasserstoff bedeutet, $R_2$ Carbamoyl, N-$C_1$-$C_4$-Alkylcarbamoyl, wie N-Methylcarbamoyl, oder N,N-Di-$C_1$-$C_4$-alkylcarbamoyl, wie N,N-Dimethylcarbamoyl, bedeutet und $R_3$ für Wasserstoff steht, vorzugsweise solche, in denen $R_2$ Carbamoyl darstellt.

Die Erfindung betrifft insbesondere andererseits Verbindungen der Formel I, worin Ph 2,6-Dihalogenphenyl, wie 2,6-Difluorphenyl, bedeutet, wobei Halogen jeweils eine Atomnummer bis und mit 35 aufweisen kann, alk für Methylen steht, $R_1$ Carbamoyl, N-$C_1$-$C_4$-Alkylcarbamoyl, wie N-Methylcarbamoyl, oder N,N-Di-$C_1$-$C_4$-alkylcarbamoyl, wie N,N-Dimethylcarbamoyl, bedeutet, $R_2$ Wasserstoff, $C_1$-$C_4$-Alkyl, wie Methyl, Carbamoyl oder mit $R_1$ identisches N-$C_1$-$C_4$-Alkylcarbamoyl, wie N-Methylcarbamoyl, oder N,N-Di-$C_1$-$C_4$-alkylcarbamoyl, wie N,N-Dimethylcarbamoyl, darstellt und $R_3$ für Wasserstoff steht, vorzugsweise solche, in denen entweder $R_1$ und $R_2$ beide für Carbamoyl stehen, oder $R_1$ Wasserstoff und $R_2$ Carbamoyl, N-$C_1$-$C_4$-Alkylcarbamoyl oder N,N-Di-$C_1$-$C_4$-alkylcarbamoyl bedeutet oder $R_1$ für Carbamoyl oder N-$C_1$-$C_4$-Alkylcarbamoyl steht und $R_2$ Wasserstoff ist.

Die Erfindung betrifft in allererster Linie Verbindungen der Formel I, worin Ph o-$C_1$-$C_4$-Alkylphenyl, wie o-Methylphenyl, o-Halogenphenyl, wie o-Fluor- oder o-Chlorphenyl, oder 2,6-Dihalogenphenyl, wie 2,6-Difluorphenyl, ferner 2,4-Dihalogenphenyl, wie 2,4-Difluorphenyl, oder 2,5-Dihalogenphenyl, wie 2,5-Difluor-oder
2-Fluor-5-chlor-phenyl, bedeutet, wobei Halogen jeweils eine Atomnummer bis und mit 35 aufweisen kann, alk Methylen darstellt, $R_1$ Wasserstoff bedeutet, $R_2$ für Carbamoyl steht und $R_3$ Wasserstoff darstellt.

Die Erfindung betrifft vorzugsweise die in den Beispielen genannten Verbindungen der Formel I.

Das erfindungsgemässe Verfahren zur Herstellung der Verbindungen der Formel I beruht auf an sich bekannten Verfahrensweisen. Es ist dadurch gekennzeichnet, dass man

a) in eine Verbindung der Formel

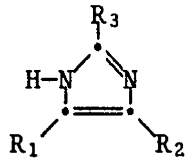

(II),

oder ein Salz davon den Rest der Formel Ph-alk- einführt oder

b) in einer Verbindung der Formel

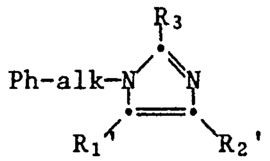

(III),

worin $R_1$' einen Rest $R_1$ oder X und $R_2$' einen Rest X oder $R_1$' einen Rest X und $R_2$' einen Rest $R_2$ darstellt, wobei X eine in gegebenenfalls ein- oder zweifach durch $C_1$-$C_7$-Alkyl substituiertes Carbamoyl überführbare Gruppe bedeutet, den Rest X bzw. die Reste X in gegebenenfalls ein- oder zweifach durch $C_1$-$C_7$-Alkyl substituiertes Carbamoyl überführt oder

c) zur Herstellung von Verbindungen der Formel I, worin $R_2$ di-$C_1$-$C_4$-alkyliertes Carbamoyl und $R_3$ Waserstoff darstellt, eine Verbindung der Formel

Ph-alk-$NH_2$ (IV)

mit einem Isonitril der Formel

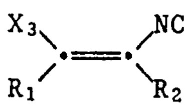

(V),

worin $X_3$ eine nukleofuge Abgangsgruppe darstellt, kondensiert und gewünschtenfalls jeweils ein gegebenenfalls erhaltenes Isomerengemisch in die Komponenten auftrennt und das gewünschte Isomere der Formel I isoliert, eine verfahrensgemäss erhältliche Verbindung der Formel I in eine andere Verbindung der Formel I überführt und/oder ein verfahrensgemäss erhältliches Diastereomeren- oder Enantiomerengemisch in die Diastereomeren oder Enantiomeren auftrennt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensationsmittel, Solvolysemittel und/oder Lösungs- bzw. Verdünnungsmittel, und Reaktions-, wie Temperatur- und Druckbedingungen, sowie gegebenenfalls Schutzgase angewendet.

Die Einführung des Restes der Formel Ph-alk- in Verbindungen II gemäss Verfahrensvariante a) erfolgt beispielsweise durch Umsetzung mit einer Verbindung der Formel Ph-alk-$X_4$ (VI), worin $X_4$ reaktionsfähiges verestertes Hydroxy, vorzugsweise mit einer Mineralsäure oder einer organischen Sulfonsäure, wie mit einer Halogenwasserstoffsäure oder einer aliphatischen oder aromatischen Sulfonsäure,verestertes Hydroxy, z.B. Chlor, Brom, Iod oder Methan-, Aethan-, Benzol- oder p-Toluolsulfonyloxy, ist.

Die Umsetzung erfolgt beispielsweise unter basischen Bedingungen, wie in Gegenwart eines basischen Kondensationsmitttels,oder indem man die Komponente II als Salz einsetzt, erforderlichenfalls unter Erwärmen, vorzugsweise in einem Lösungs- oder Verdünnungsmittel. Basische Kondensationsmittel sind beispielsweise mit der Komponente II salzbildende basische Kondensationsmittel, wie Alkalimetallalkoholate, z.B. Natriummethanolat, Kaliumtertiärbutanolat oder Natriumäthanolat, oder Alkalimetall- oder Erdalkalimetallamide, z.B. Natriumamid oder Lithiumdiisopropylamid. Die Ueberführung der Komponente II in eines ihrer

Salze wird vorteilhaft vorab durchgeführt, z.B. durch Umsetzung mit einer der genannten Basen. Lösungsmittel sind bei der Durchführung der Umsetzung in Gegenwart eines Alkoholates vorzugsweise die entsprechenden Alkohole und bei der Durchführung in Gegenwart eines Metallamides beispielsweise aprotische organische Lösungsmittel, wie N-Niederalkylalkansäureamide, Phosphorsäureniederalkylamide oder Diniederalkylsulfoxide, z.B. N,N-Dimethylformamid, Hexamethylphosphorsäuretriamid oder Dimethylsulfoxid. Die Reaktion verläuft vorzugsweise in der Wärme, z.B. im Temperaturbereich von etwa 20-150 ° C, vorzugsweise von etwa 25-100 ° C.

Ausgehend von Verbindungen II, worin $R_1$ und $R_2$ verschieden sind, können Gemische der beiden möglichen Isomeren erhalten werden, die zur Isolierung nur eines Isomeren aufgetrennt werden müssen.

Die Verfahrensvariante a) eignet sich deshalb insbesondere für die Herstellung von Verbindungen, worin $R_1$ und $R_2$ identische,gegebenenfalls mono- oder dialkylierte, Carbamoylgruppen bedeuten.

Ausgangsstoffe II können beispielsweise hergestellt werden, indem man in einer Verbindung der Formel

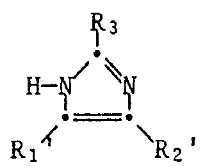 (VII),

worin einer der Reste $R_1'$ und $R_2'$ eine Halogencarbonyl- oder Niederalkoxycarbonylgruppe und der andere Wasserstoff, $C_1$-$C_7$-Alkyl oder eine Halogencarbonyl- oder Niederalkoxycarbonylgrupe bedeutet, die Halogencarbonyl- bzw. Niederalkoxycarbonylgruppe(n)$R_1'$ und/oder $R_2'$ durch Umsetzung mit Ammoniak oder einem Mono- oder Dialkylamin in gegebenenfalls mono- oder dialkyliertes Carbamoyl $R_1$ und/oder $R_2$ überführt.

Verbindungen VII ihrerseits können hergestellt werden, indem man Imidazol-4,5-dicarbonsäure , erforderlichenfalls in Gegenwart katalytischer Mengen von N,N-Dimethylformamid oder Pyridin, mit Thionylchlorid zum 4,5-Dicarbonsäurechlorid oder in Gegenwart katalytischer Mengen von Mineralsäuren, z.B. von Chlorwasserstoffsäure oder Schwefelsäure, mit einem Niederalkanol zu einem 4,5-Dicarbonsäureniederalkylester umsetzt.

Gemäss der Verfahrensvariante b) in gegebenenfalls mono- oder dialkylierte Carbamoylreste überführbare Gruppen X sind beispielsweise freie, veresterte oder in einer Salz- oder Anhydridform vorliegende Carboxygruppen, die Cyanogruppe oder gegebenenfalls alkylierte Amidinogruppen.

Veresterte Carboxygruppen sind beispielsweise mit einem Niederalkanol oder einem Niederalkylmercaptan veresterte Carboxygruppen, d.h. Niederalkoxy- oder Niederalkylthiocarbonylgruppen, können aber auch mit einem beliebigen anderen Alkohol oder Mercaptan, z.B. mit einem gegebenenfalls substituierten Phenol, verestert sein.

In Salzform vorliegende Carboxygruppen sind beispielsweise in einer von Ammoniak oder einem Mono- oder Diniederalkylamin abgeleiteten Ammoniumsalzform vorliegende Carboxygruppen, ferner in Metall-, z.B. Alkali- oder Erdalkalimetallsalzform vorliegende Carboxygruppen.

In Anhydridform vorliegende Carboxygruppen sind beispielsweise in einer Halogenidform vorliegende Carboxygruppen, wie Chlorcarbonyl, können aber auch mit einer reaktiven Carbonsäure anhydridisiert sein und beispielsweise Alkoxycarbonyloxycarbonyl oder Trifluoracetoxycarbonyl bedeuten.

Gegebenenfalls alkylierte Amidinogruppen sind beispielsweise unsubstituierte oder N-alkylierte oder N,N-dialkylierte Amidinogruppen.

Die Ueberführung der Gruppe(n) X in gegebenenfalls wie angegeben substituiertes Carbamoyl $R_1$ und/oder $R_2$ erfolgt in üblicher Weise, z.B. durch Solvolyse, d.h. Hydrolyse oder Ammono- bzw. Aminolyse (Umsetzung mit Wasser oder Ammoniak bzw. einem Mono- oder Dialkylamin).

Durch Hydrolyse kann man beispielsweise unsubstituierte oder N-mono- oder N,N-dialkylierte Amidinogruppen X in gegebenenfalls alkylierte lierte Carbamoylgruppen bzw. Cyano X in Carbamoyl überführen. Die Hydrolyse der genannten Amidinogruppen erfolgt beispielsweise unter sauren Bedingungen, z.B. in Gegenwart von Mineralsäuren, z.B. von Salz- oder Schwefelsäure. Die Hydrolyse von Cyano wird beispielsweise in Gegenwart eines basischen Hydrolysemittels, wie eines Alkalimetallhydroxides, z.B. von Natronlauge oder Kalilauge,durchgeführt. Die Hydrolyse kann durch Peroxoverbindungen, z.B. Wasserstoffperoxid,erleichtert werden und erfolgt beispielsweise in einem Niederalkanol, z.B. in Aethanol, als Verdünnungsmittel.

Durch Ammono- bzw. Aminolyse kann man beispielsweise freie oder veresterte oder in einer Salz- oder Anhydridform vorliegende Carboxygruppen X in unsubstituiertes oder alkyliertes Carbamoyl überführen.

Dabei arbeitet man erforderlichenfalls in Gegenwart eines Kondensationsmittels,vorteilhaft in einem inerten Lösungsmittel. Kondensationsmittel sind beispielsweise basische Kondensationsmittel, vor allem Ammoniak bzw. für die Aminolyse verwendete Amine im Ueberschuss,ausgehend von Verbindungen III, worin X in Anhydridform vorliegendes Carboxy bedeutet, ferner Alkalimetallhydroxide oder -carbonate, oder tertiäre organische Stickstoffbasen, wie Triniederalkylamine oder tertiäre heteroaromatische Stickstoffbasen, z.B. Triäthylamin oder Pyridin. Als inertes Lösungsmitel ist beispielsweise Toluol, bei der Solvolyse von veresterten Carboxygruppen ferner Aethanol,besonders geeignet. Freie Carboxygruppen können unter Dehydratisierung der intermediär gebildeten Ammoniumsalze, z.B. durch Erhitzen oder Einwirkung wasserentziehender Mittel, wie von Säureanhydriden, z.B. von Phosphorpentoxid, Acetylchlorid und dergleichen, oder von Carbodiimiden, z.B. von N,N'-Dicyclohexylcarbodiimid, in gegebenenfalls alkyliertes Carbamoyl überführt werden.

Die Ausgangsstoffe der Formel III können, soweit sie nicht bekannt sind, in üblicher Weise hergestellt werden, beispielsweise durch Umsetzung einer Verbindung der Formel

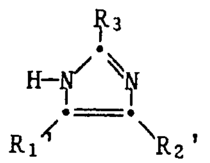

(XI)

mit einer Verbindung der Formel Ph-alk-$X_4$ (VI), worin $X_4$ reaktionsfähiges verestertes Hydroxy, z.B. Chlor, Brom, Iod oder Methan- oder p-Toluolsulfonyloxy, bedeutet. Die Umsetzung von Verbindungen XI und VI erfolgt insbesondere in analoger Weise wie unter der Verfahrensvariante a) beschrieben. Verfahrensgemäss erhältliche Verbindungen III, worin $R_1'$ und/oder $R_2'$ verestertes Carboxy oder Cyano bedeuten, können nachfolgend, z.B. durch Behandeln mit Natrium- oder Kaliumhydroxid in wässrig-äthanolischer Lösung, zu den entsprechenden Carbonsäuren (III; $R_1'$ und/oder $R_2'$ = Carboxy) hydrolysiert werden, die in einem weiteren Reaktionsschritt, z.B. mittels Thionylchlorid in Toluol/Pyridin oder Phosphorpentachlorid in Tetrahydrofuran bzw. mittels Phosphoroxychlorid, in die Säurechloride (III; $R_1'$ und/oder $R_2'$ = Chlorcarbonyl) überführt werden können. Primär erhaltene Nitrile (III; $R_1'$ und/oder $R_2'$ = Cyano) können auch durch Umsetzung mit einer Mineralsäure, z.B. mit Chlor- oder Bromwasserstoffsäure oder Schwefelsäure und einem Alkohol, z.B. einem Niederalkanol oder Benzylalkohol,und Weiterumsetzung des gebildeten Iminoäthers mit Ammoniak oder einem Mono-oder Dialkylamin in die entsprechenden Amidine (III; $R_1'$ und/oder $R_2'$ = gebenenfalls alkyliertes Amidino) umgewandelt werden.

Gemäss einer alternativen Verfahrensweise erhält man Verbindungen III, indem man eine Verbindung der Formel Ph-C(=O)-R (VIII), worin R Wasserstoff oder $C_1$-$C_3$-Alkyl ist, z.B. in Gegenwart von Schwefelsäure in siedendem Aethanol oder Tetrahydrofuran, mit einer Verbindung der Formel $H_2N$-C($R_1'$)=C($R_2'$)-$NH_2$ (IX) umsetzt, in dem Reaktionsprodukt der Formel $H_2N$-C($R_1'$)=C($R_2'$)-N=C(R)-Ph (X) bzw. $H_2N$-C-($R_2'$)=C($R_1'$)-N=C(R)-Ph (IIIe) die C-N-Doppelbindung, z.B. mit Natriumborhydrid in Methanol/Tetrahydrofuran, zur Einfachbindung reduziert und das Reaktionsprodukt, z.B. in siedendem Diäthylenglykoldimethyläther,mit Ameisensäure oder in Gegenwart eines sauren Katalysators mit einem Orthoameisensäureester, z.B. bei etwa 80-100 °C mit Orthoameisensäuretriäthylester, kondensiert. Diese Verfahrensweise eignet sich besonders zur Herstellung von Dinitrilen (III, $R_1'$ = $R_2'$ = Cyano), die gewünschtenfalls wie vorstehend beschrieben zu entsprechenden Dicarbonsäuren (III; $R_1'$ = $R_2'$ = Carboxy) hydrolysiert und gegebenenfalls nachfolgend verestert oder anhydridisiert bzw. in ein entsprechendes Bis-Amidin III, worin $R_1'$ und $R_2'$ jeweils für eine gegebenenfalls N-mono- oder N,N-dialkylierte Amidinogruppe stehen, überführt werden können.

In Ausgangsstoffen V für die Verfahrensvariante c) sind nukleofuge Abgangsgruppen $X_3$ beispielsweise disubstituierte Aminogruppen, wie Diniederalkylamino, z.B. Dimethylamino, oder 5- bis 7-gliedriges Alkylenamino, z.B. Pyrrolidino oder Piperidino, bzw. Aza, Oxa- oder Thiaalkylenamino, z.B. N'-Methylpiperazino, Morpholino oder Thiomorpholino.

Die Umsetzung von Verbindungen IV und V erfolgt in üblicher Weise, beispielsweise durch Erwärmen in einem inerten Lösungsmittel, wie einem aromatischen oder araliphatischen Kohlenwasserstoff, z.B. in Benzol, Toluol oder Xylol, im Temperaturbereich von etwa 80 bis 180 °C, vorzugsweise von etwa 110-150 °C.

Ausgangsstoffe IV können beispielsweise durch Umsetzung von Verbindungen der Formel Ph-alk-$X_4$ - (VI; $X_4$ = Chlor, Brom oder Iod) mit Ammoniak unter basischen Bedingungen oder durch Reduktion entsprechender Nitrile der Formel Ph-CN (XIII), beispielsweise mittels eines Dileichtmetallhydrides, z.B.

mittels Lithiumaluminiumhydrid, hergestellt werden.

Ausgangsstoffe V werden beispielsweise erhalten, indem man einen 2-Isocyanoessigsäureniederalkylester durch Behandeln mit einem N,N-Di-$C_1$-$C_4$-alkylamin in das entsprechende 2-Isocyano-N,N-di-$C_1$-$C_4$-alkylacetamid überführt und dieses mit einem N,N-disubstituierten Formamidacetal, beispielsweise mit einem N,N-Diniederalkyl- oder einem 5- bis 7-gliedrigen N,N-Alkylen- bzw. N,N-Aza-, N,N-Oxa- oder N,N-Thiaalkylen-formamiddiniederalkylacetal, z.B. mit N,N-Dimethylformamiddimethylacetal, umsetzt.

Einem besonders eleganten Verfahren zur Herstellung von Verbindungen I, worin $R_1$ Wasserstoff oder Niederalkyl und $R_3$ Wasserstoff bedeutet, zufolge setzt man eine Verbindung der Formel Ph-alk-$NH_2$ (IV) in einem inerten Lösungsmittel mit einem entsprechenden Isonitril der Formel CN-C($R_2$')=C($R_1$)-$X_3$ (XII), worin $R_2$' N,N-Di-$C_1$-$C_4$-alkylcarbamoyl oder verestertes Carboxy bedeutet, zu einer Verbindung I, worin $R_1$ Wasserstoff oder Niederalkyl, $R_2$ N,N-Di-$C_1$-$C_4$-alkylcarbamoyl und $R_3$ Wasserstoff ist, bzw. zu einer Verbindung III, worin $R_1$' Wasserstoff oder Niederalkyl, $R_2$' verestertes Carboxy und $R_3$ Wasserstoff ist, um. Letztere wird anschließend durch Umsetzung mit Ammoniak oder einem N-$C_1$-$C_7$-Alkylamin gemäß Verfahrensvariante b) in die entsprechende Verbindung I, worin $R_2$ Carbamoyl oder N-$C_1$-$C_7$-Alkylcarbamoyl ist, überführt. Geeignete Gruppen $X_3$ sind beispielsweise die für die Verfahrensvariante c) angegebenen Dimethylamino oder 5- bis 7-gliedriges Alkylenamino, z.B. Pyrrolidino oder Piperidino, bzw. Aza-, Oxa- oder Thiaalkylenamino, z.B. N'-Methylpiperazino, Morpholino oder Thiomorpholino. Vorzugsweise arbeitet man unter Erwärmen, z.B. im Temperaturbereich von etwa 80-180 °C, z.B. in der Siedehitze.

Verfahrensgemäß erhältliche Verbindungen I können in andere Verbindungen der Formel I überführt werden, indem man eine oder mehrere Variablen der allgemeinen Formel I in andere überführt.

So kann man unsubstituiertes Carbamoyl $R_2$ und gegebenenfalls $R_1$ in N-Mono- oder N,N-Di-$C_1$-$C_7$-alkylcarbamoyl, ebenso N-Mono-$C_1$-$C_7$-alkylcarbamoyl $R_2$ und gegebenenfalls $R_1$ in N,N-Di-$C_1$-$C_7$-alkylcarbamoyl überführen, beispielsweise durch Behandeln mit einem reaktiven Ester eines $C_1$-$C_7$-Alkanols, wie einem $C_1$-$C_7$-Alkylhalogenid, z.B. -bromid oder -iodid, $C_1$-$C_7$-Alkansulfonat, z.B. -methansulfonat, oder einem Di-$C_1$-$C_7$-alkylsulfat, z.B. Dimethylsulfat, vorzugsweise unter basischen Bedingungen,
wie in Gegenwart
von Natriumamid oder Natriumhydrid oder von Natronlauge oder Kalilauge und eines Phasentransfer-Katalysators, wie von Tetrabutylammoniumbromid oder Benzyltrimethylammoniumchlorid.

Die Verbindung I kann,je nach der Anzahl der asymmetrischen Kohlenstoffatome,in Form eines reinen optischen Isomeren (Enantiomeren oder Diastereomeren) oder als Gemisch derselben (Racemat, Diastereoisomerengemisch oder Racematgemisch ) vorliegen.

Erhaltene Isomerengemische von Imidazol-4(5)-carboxamiden und ihrer an der Amidogruppe $C_1$-$C_7$-alkylierten Derivate sowie verfahrensgemäss erhältliche Diastereomerengemische und Racematgemische können auf Grund der physikalisch-chemischen Unterschiede der Bestandteile in bekannter Weise in die reinen Stellungsisomeren, Diastereomeren und Racemate aufgetrennt werden, beispielsweise durch Chromatographie und/oder fraktionierte Kristallisation. Erhaltene Enantiomerengemische, z.B. Racemate, lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Vorproduktes, z.B. der Formel III (X = Carboxy), mit einer mit der racemischen Säure Salze bildenden optisch aktiven Base und Trennung der auf diese Weise erhaltenen diastereomeren Salze, z.B. auf Grund ihrer verschiedenen Löslichkeiten, in die Diastereromeren, aus denen die Antipoden durch Einwirkung geeigneter Mittel freigesetzt werden können . Geeignete optisch aktive Basen sind beispielsweise entsprechende Alkaloide, wie Brucin, Strychnin, Ephedrin, Chinin, Chinidin oder Andronin, ebenso Menthylamin oder $\alpha$-Phenyläthylamin.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Neue Ausgangsstoffe, die speziell für die Herstellung der erfindungsgemässen Verbindungen entwickelt wurden, insbesondere die zu den eingangs als bevorzugt gekennzeichneten Verbindungen der Formel I führende Ausgangsstoffauswahl, die Verfahren zu ihrer Herstellung sowie ihre Verwendung als Zwischenprodukte bilden ebenfalls einen Gegenstand der Erfindung.

Alle vorstehend erwähnten Verbindungen der Formel I, mit Ausnahme derjenigen, in denen Ph-alk Monohalogenphenyl-$C_1$-$C_2$-alkyl, $R_3$ $C_1$-$C_7$-Alkyl, einer der Reste $R_1$ und $R_2$ Carbamoyl und der andere der Reste $R_1$ und $R_2$ Wasserstoff ist, können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen,enthalten, die sich zur enteralen, z.B. oralen, oder parenteralen Verabreichung eignen. So verwendet man Tabletten oder

Gelatinekapseln, welche den Wirkstoff zusammen mit Verdünnungsmitteln, z.B. Laktose, Dextrose, Saccharose, Mannitol, Sorbitol, Cellulose und/oder Glycin, und/oder Schmiermitteln,z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel aufweisen. Ferner kann man die Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer, enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 %, des Aktivstoffes.

Die Erfindung betrifft ebenfalls die Verwendung aller vorstehend erwähnten Verbindungen der Formel I, mit Ausnahme derjenigen, in denen Ph-alk Monohalogenphenyl-$C_1$-$C_2$-alkyl, $R_3$ $C_1$-$C_7$-Alkyl, einer der Reste $R_1$ und $R_2$ Carbamoyl und der andere der Reste $R_1$ und $R_2$ Wasserstoff ist, vorzugsweise in Form von pharmazeutischen Präparaten. Die Dosierung kann von verschiedenen Faktoren, wie Applikationsweise, Spezies, Alter und/oder individuellem Zustand, abhängen. Die täglich zu verabreichenden Dosen liegen bei oraler Applikation zwischen etwa 5 und etwa 50 mg/kg und für Warmblüter mit einem Gewicht von etwa 70 kg vorzugsweise zwischen etwa 0,5 g und etwa 5,0 g.

Die folgenden Beispiele dienen zur Illustration der Erfindung; Temperaturen werden in Celsiusgraden angegeben.

Beispiel 1: 5,34 g 1-(2,3-Difluorbenzyl)imidazol-4-carbonsäureäthylester werden in 80 ml Methanol gelöst und in einen Autoklaven überführt. Man presst 20,0 g Ammoniak auf und erhitzt 19 Stunden auf 100°. Nach Abblasen des überschüssigen Ammoniaks wird das ausgefallene Produkt abfiltriert und aus Aethanol umkristallisiert. Man erhält 1-(2,3-Difluorbenzyl)imidazol-4-carboxamid vom Smp. 221-222°.

In analoger Weise erhält man

1-(2,4-Difluorbenzyl)imidazol-4-carboxamid, Smp. 220-221°;
1-(2,5-Difluorbenzyl)imidazol-4-carboxamid, Smp. 214-215° (aus Dioxan);
1-(2,6-Difluorbenzyl)imidazol-4-carboxamid, Smp. 227-229° (aus Aethanol);
1-(2,3-Difluorbenzyl)imidazol-5-carboxamid, Smp. 199-200° (aus Isopropanol);
1-(2,4-Difluorbenzyl)imidazol-5-carboxamid, Smp. 207-208° (aus Isopropanol);
1-(2,5-Difluorbenzyl)imidazol-5-carboxamid, Smp. 211-212° (aus Isopropanol);
1-(2,6-Difluorbenzyl)imidazol-5-carboxamid, Smp. 184-185° (aus Methanol);
1-(2,6-Difluorbenzyl)imidazol-4,5-dicarboxamid, Smp. 218-220° (aus Dioxan/Aether);
1-(o-Chlorbenzyl)imidazol-4,5-dicarboxamid, Smp. 237-238° (aus Essigsäure/Wasser);
1-(o-Methylbenzyl)imidazol-4,5-dicarboxamid, Smp. 219-221° (aus Dioxan);
1-(2,6-Difluorbenzyl)-2-methyl-imidazol-4,5-dicarboxamid, Smp. über 300° (aus Essigsäure/Wasser);
1-(2,6-Difluorbenzyl)-4-methyl-imidazol-5-carboxamid, Smp. 233-235°;
1-(o-Chlorbenzyl)-4-methyl-imidazol-5-carboxamid, Smp. 252-254°;
1-(2,6-Difluorbenzyl)-5-methyl-imidazol-4-carboxamid, Smp. 235-236°;
1-(o-Chlorbenzyl)-5-methyl-imidazol-4-carboxamid, Smp. 193-195°;
1-(2,6-Difluorbenzyl)-2-methyl-imidazol-4-carboxamid, Smp. 266-267°;
1-[1-(2,6-Difluorphenyl)äthyl]imidazol-4-carboxamid, Smp. 220°, und
1-[1-(2,6-Difluorphenyl)äthyl]imidazol-5-carboxamid, Smp. 163-165°.

Die Ausgangsstoffe können z.B. folgendermassen hergestellt werden:

a) 1,32 g Natrium werden in 150 ml Aethanol gelöst. Man gibt 6,72 g Imidazol-4-carbonsäureäthylester und 9,9 g 2,3-Difluorbenzylbromid hinzu und erhitzt 3,5 Stunden unter Rühren zum Rückfluss. Das Reaktionsgemisch wird unter vermindertem Druck eingedampft, der Eindampfrückstand mit Eiswasser verrührt und dreimal mit je 50 ml Essigsäureäthylester ausgeschüttelt. Die Auszüge werden vereinigt, mit Wasser und mit gesättigter Kochsalzlösung gewaschen, über Magnesiumsulfat getrocknet und unter vermindertem Druck zur Trockne eingedampft. Man erhält 1-(2,3-Difluorbenzyl)imidazol-4(5)-carbonsäureäthylester als Isomerengemisch, das durch Chromatographie an Kieselgel als stationärer und

Toluol/Isopropanol (9:1) als mobiler Phase in die Komponenten, 1-(2,3-Difluorbenzyl)-imidazol-4-carbonsäureäthylester, Smp. 95-100° (aus Toluol/Hexan),und 1-(2,3-Difluorbenzyl)imidazol-5-carbonsäureäthylester(Oel), aufgetrennt werden kann.

b) 13,0 g 2,6-Difluorbenzylalkohol und 8,4 g Methansulfonylchlorid werden in 90 ml Methylenchlorid gelöst und unter Rühren im Eisbad tropfenweise mit 17,6 ml Triäthylamin in 18 ml Methylenchlorid versetzt. Die Eintropfgeschwindigkeit wird so einreguliert, dass die Innentemperatur 10° nicht überschreitet. Man lässt 40 Minuten im Eisbad nachrühren, versetzt mit 90 ml Eiswasser und lässt weitere 60 Minuten rühren. Die organische Phase wird abgetrennt, über Magnesiumsulfat getrocknet und mit 21 g des Mononatriumsalzes von Imidazol-4,5-dicarbonsäurediäthylester (erhältlich z.B. durch Umsetzung von Imidazol-4,5-dicarbonsäurediäthylester mit 2,53 g Natrium, gelöst in 60 ml Aethanol) versetzt. Man lässt 3 Stunden bei Raumtemperatur rühren, wäscht dreimal mit Wasser, trocknet über Magnesiumsulfat, dampft zur Trockne ein, löst in Toluol/Essigester (1:1), filtriert über Kieselgel, dampft erneut zur Trockne ein und kristallisiert aus Aether/Petroläther um. Man erhält den 1-(2,6-Difluorbenzyl)imidazol-4,5-dicarbonsäurediäthylester vom Smp. 68-70°.

In analoger Weise erhält man:

1-(2,4-Difluorbenzyl)imidazol-4-carbonsäureäthylester, Smp. 89-90°, und 1-(2,4-Difluorbenzyl)imidazol-5-carbonsäureäthylester, Smp. 70-72°;

1-(2,5-Difluorbenzyl)imidazol-4-carbonsäureäthylester, Smp. 138-139°, und 1-(2,5-Difluorbenzyl)imidazol-5-carbonsäureäthylester, Smp. 74-75°;

1-(2,6-Difluorbenzyl)imidazol-4-carbonsäureäthylester, Smp. 114-115°, und 1-(2,6-Difluorbenzyl)imidazol-5-carbonsäureäthylester` Smp. 63-64°;

ausgehend von 4-Methylimidazol-5-carbonsäureäthylester 1-(2,6-Difluorbenzyl)4-methyl-imidazol-5-carbonsäureäthylester, Smp. 59-61° (aus Hexan) und 1-(2,6-Difluorbenzyl)-5-methyl-imidazol-4-carbonsäureäthylester` Smp. 107-108° (aus Toluol/Aether); 1-(o-Chlorbenzyl)-4-methyl-imidazol-5-carbonsäureäthylester, Smp. 74-76°, und 1-(o-Chlorbenzyl)-5-methyl-imidazol-4-carbonsäureäthylester, Smp. 100-104°;

ausgehend von Imidazol-4,5-dicarbonsäurediäthylester 1-(2,6-Difluorbenzyl)imidazol-4,5-dicarbonsäurediäthylester, Smp. 71-73° (aus Petroläther), 1-(o-Chlorbenzyl)imidazol-4,5-dicarbonsäurediäthylester, Smp. 69-10° (aus Aether/Hexan), und 1-(o-Methylbenzyl)imidazol-4,5-dicarbonsäurediäthylester (Oel); sowie ausgehend von 2-Methylimidazol-4,5-dicarbonsäurediäthylester 1-(2,6-Difluorbenzyl)-2-methyl-imidazol-4,5-dicarbonsäurediäthylester, Smp. 63-64° (aus Aether/Hexan).

c) 16,37 g 1-(2,6-Difluorphenyl)äthanol werden in 74 ml Hexan gelöst, mit 74 ml 48%iger Bromwasserstoffsäure versetzt und 90 Minuten unter Rühren zum Rückfluss erhitzt. Man lässt abkühlen, trennt die organische Phase ab und schüttelt die wässrige Phase 3-mal mit Hexan aus. Die vereinigten Hexanphasen werden über Magnesiumsulfat getrocknet und filtriert. Die erhaltene Lösung von 1-(2,6-Difluorphenyl)äthylbromid wird zu einer zuvor mit einer Lösung von 2,39 g Natrium in 50 ml Aethanol versetzter Lösung von 14,5 g Imidazol-4(5)-carbonsäureäthylester in 100 ml Aethanol hinzugegeben. Man destilliert das Hexan ab, erwärmt anschliessend 3 Stunden zum Rückfluss und erhält nach Aufarbeitung analog zu 1a) 1-[1-(2,6-Difluorphenyl)äthyl]imidazol-4-carbonsäureäthylester, Smp. 96-97°, sowie 1-[1-(2,6-Difluorphenyl)äthyl]-imidazol-5-carbonsäureäthylester, Smp. 106-107°.

Beispiel 2: Durch Umsetzung von 1-(2,6-Difluorbenzyl)imidazol-4-carbonsäureäthylester mit der entsprechenden Menge Methylamin bzw. Dimethylamin in analoger Weise wie in Beispiel 1 beschrieben erhält man 1-(2,6-Difluorbenzyl)imidazol-4-(N-methyl)carboxamid, Smp. 155-157° (aus Essigester),und 1-(2,6-Difluorbenzyl)imidazol-4-(N,N-dimethyl)carboxamid, Smp. 113-115° (aus Essigester/Hexan), ferner durch Umsetzung von 1-(2,6-Difluorbenzyl)imidazol-4-carbonsäureäthylester mit der entsprechenden Menge Aethylamin 1-(2,6-Difluorbenzyl)imidazol-4-(N-äthyl)carboxamid, Smp. 112-114°, sowie durch Umsetzung von 1-(2,6-Difluorbenzyl)imidazol-5-carbonsäureäthylestermit Methylamin 1-(2,6-Difluorbenzyl)imidazol-5-(N-methyl)-carboxamid, Smp. 128-130°.

Beispiel 3: 4,0 g 1-(o-Fluorbenzyl)imidazol-4-carbonsäuremethylester werden in 80 ml Methanol gelöst und in einen Autoklaven überführt. Man presst 20,0 g Ammoniak auf und erhitzt 19 Stunden auf 100°. Nach Abblasen des überschüssigen Ammoniaks wird das ausgefallene Produkt abfiltriert und aus Aethanol umkristallisiert. Man erhält 1-(o-Fluorbenzyl)imidazol-4-carboxamid vom Smp. 204-205°.

In analoger Weise erhält man:

1-(2,6-Difluorbenzyl)imidazol-4-carboxamid, Smp. 227-229° (Methanol);

1-(o-Chlorbenzyl)imidazol-4-carboxamid, Smp. 238-239°;

1-(o-Methylbenzyl)imidazol-4-carboxamid, Smp. 245-245,5°,und

1-(3,4-Difluorbenzyl)imidazol-4-carboxamid, Smp. 184-185°.

Die Ausgangsstoffe können z.B. folgendermassen hergestellt werden:

8 g o-Fluorbenzylamin und 6,6 g 2-Isocyano-3-dimethylamino-acrylsäuremethylester werden in 66 ml Xylol gelöst und 3 Stunden unter Rühren zum Rückfluss erhitzt. Nach Abkühlen auf Raumtemperatur wird mit Essigester verdünnt und zweimal mit je 50 ml 2n-Salzsäure und anschliessend mit Wasser ausgeschüttelt. Die wässrigen Phasen werden zweimal mit je 50 ml Essigester gewaschen. Sämtliche wässrigen Phasen werden vereinigt, mit konzentriertem Ammoniak alkalisch gestellt und dreimal mit je 100 ml Dichlormethan ausgeschüttelt. Die Auszüge werden vereinigt, zweimal mit Wasser gewaschen, über Magnesiumsulfat getrocknet und zur Trockne eingedampft. Der Eindampfrückstand wird in Essigester gelöst, über Kieselgel filtriert, eingeengt und durch Hinzufügen von Diäthyläther zur Kristallisation gebracht. Man erhält 1-(o-Fluorbenzyl)imidazol-4-carbonsäuremethylester vom Smp. 87-89°.

In analoger Weise erhält man:
1-(2,6-Difluorbenzyl)imidazol-4-carbonsäuremethylester, Smp. 107-109°;
1-(o-Chlorbenzyl)imidazol-4-carbonsäuremethylester, Smp. 82-83°;
1-(o-Methylbenzyl)imidazol-4-carbonsäuremethylester, Smp. 89-90°, und
1-(3,4-Difluorbenzyl)imidazol-4-carbonsäuremethylester, Smp. 139-141°.

Beispiel 4: 15,7 g 2,6-Difluorbenzylamin und 11,2 g 2-Isocyano-3-dimethylamino-acrylsäure(N,N-dimethyl)amid werden in 150 ml Toluol gelöst und 3 Stunden unter Rühren zum Rückfluss erhitzt. Nach Abkühlen auf Raumtemperatur wird mit Essigester verdünnt und zweimal mit je 50 ml 2n-Salzsäure und anschliessend mit Wasser ausgeschüttelt. Die wässrigen Phasen werden zweimal mit je 50 ml Essigester gewaschen. Sämtliche wässrigen Phasen werden vereinigt, mit Ammoniak alkalisch gestellt und dreimal mit je 100 ml Trichlormethan (Chloroform) ausgeschüttelt. Die Auszüge werden vereinigt, zweimal mit Wasser gewaschen, über Magnesiumsulfat getrocknet und zur Trockne eingedampft. Der Eindampfrückstand wird in Essigester gelöst, über Kieselgel filtriert, eingeengt und durch Hinzufügen von Diäthyläther zur Kristallisation gebracht. Man erhält 1-(2,6-Difluorbenzyl)imidazol-4-(N,N-dimethyl)carboxamid vom Smp. 113-115° (aus Aether/Hexan).

Beispiel 5: 0,29 g Natrium werden in 30 ml Aethanol gelöst. Man fügt 1,9 g Imidazol-4,5-di(N-methylcarboxamid) und 2,4 g 2,6-Difluorbenzylbromidhinzu und erhitzt 5 Stunden unter Rühren zum Rückfluss. Das Reaktionsgemisch wird zur Trockene eingedampft, der Rückstand in Methylenchlorid aufgenommen, mit Wasser ausgeschüttelt, über Magnesiumsulfat getrocknet und eingedampft. Der Eindampfrückstand wird in Toluol/Essigester (1:1) gelöst, über Kieselgel filtriert, erneut eingedampft und aus Essigester umkristallisiert. Man erhält 1-(2,6-Difluorbenzyl)-imidazol-4,5-di(N-methylcarboxamid) vom Smp. 112-113°. In analoger Weise erhält man auch 1-(2,6-Difluorbenzyl)imidazol-4,5-di(N,N-dimethylcarboxamid) vom Smp. 184-185°.

Die Ausgangsmaterialien können beispielsweise folgendermassen hergestellt werden:
6,36 g Imidazol-4,5-dicarbonsäurediäthylester werden in 140 ml Aethanol gelöst und in einen Autoklaven überführt. Man presst 45 g Dimethylamin auf und erwärmt 45 Stunden auf 100° sowie 30 Stunden auf 110°. Das Reaktionsgemisch wird zur Trockne eingedampft, an Kieselgel mit Essigester/Methanol (2:1) chromatographisch gereinigt und aus Essigester kristallisiert. Man erhält Imidazol-4,5-di(N,N-dimethylcarboxamid) vom Smp. 108-110°, analog unter Verwendung von Methylamin auch Imidazol-4,5-di(N-methylcarboxamid).

Beispiel 6: 76 mg Natrium werden in 3 ml Aethanol gelöst. Man fügt 333 mg Imidazol-4-carboxamid hinzu, lässt 30 Minuten bei Raumtemperatur rühren, gibt 64 mg 2,6-Difluorbenzylbromid, gelöst in 2 ml Aethanol, hinzu und lässt weitere 3 Stunden bei Raumtemperatur rühren. Das Reaktionsgemisch wird zur Trockne eingedampft. Man erhält ein Gemisch von 1-(2,6-Difluorbenzyl)imidazol-4 und -5-carboxamid, das durch Chromatographie an Kieselgel mit Essigester/Isopropanol (7:2) als Laufmittel in die Komponenten, 1-(2,6-Difluorbenzyl)imidazol-4-carboxamid, Smp. 227-229° (aus Aethanol). und 1-(2,6-Difluorbenzyl)imidazol-5-carboxamid, Smp. 184-185° (aus Methanol), aufgetrennt werden kann.

Beispiel 7: Zu einer Lösung von 10,0 g 1-(o-Methylbenzyl)imidazol-4,5-dinitril in 300 ml Aethanol und 25 ml 5n-Natronlauge werden bei 40-50° 25 ml 30%ige Wasserstoffperoxidlösung zugetropft. Man lässt 2 Stunden bei 40-50° rühren, verdünnt mit 500 ml Wasser und saugt ab. Nach Waschen mit Wasser und Umkristallisieren aus Dioxan erhält man 1-(o-Methylbenzyl)imidazol-4,5-dicarboxamid vom Smp. 219-221°.

Das Ausgangsmaterial kann man z.B. folgendermassen herstellen:
3,70 g Natrium werden in 375 ml Aethanol gelöst, mit 14,6 g Imidazol-4,5-dinitril und 16,5 ml o-Methylbenzylchlorid versetzt und 8 Stunden zum Rückfluss erhitzt. Man dampft unter vermindertem Druck zur Trockne ein, nimmt in Dichlormethan auf, wäscht nacheinander mit Wasser und mit gesättigter Kochsalzlösung, trocknet über Natriumsulfat und dampft zur Trockne ein. Das Rohprodukt wird an Kieselgel mit Toluol/Essigester (5:1) chromatographisch gereinigt und aus Essigester/Hexan kristallisiert. Man erhält 1-(o-Methylbenzyl)imidazol-4,5-dinitril vom Smp. 80-81°.

Beispiel 8: Tabletten, enthaltend je 50 mg 1-(2,6-Difluorbenzyl)imidazol-4-carboxamid, können wie folgt

hergestellt werden.

| Zusammensetzung (10000 Tabletten) | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Aethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 9 : Lacktabletten, enthaltend je 100 mg 1-(2,6-Difluorbenzyl)imidazol-4-carboxamid, können wie folgt hergestellt werden:

| Zusammensetzung (10000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 g |
| Schellack | 0.64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen),befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese werden mit einer Lösung der Hydroxypropylmethylcellulose und des Schellacks in Methylenchlorid lackiert; Endgewicht der Lacktablette: 283 mg.

Beispiel 10: In analoger Weise wie in den Beispielen 8 und 9 beschrieben können auch Tabletten bzw. Lacktabletten,enthaltend eine andere Verbindung gemäss einem der Beispiele 1 bis 7, hergestellt werden.

**Patentansprüche**

**Patentansprüche für folgende Vertragsstaaten : BE, CH, DE, FR, GB, IT, LI, LU, NL, SE**

**1.** Verbindungen der Formel

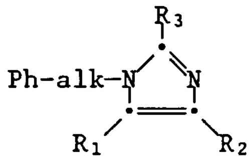

(I),

worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ für gegebenenfalls ein- oder zweifach durch $C_1$-$C_7$-Alkyl substituiertes Carbamoyl und der andere für Wasserstoff,

$C_1$-$C_7$-Alkyl oder gegebenenfalls ein- oder zweifach durch $C_1$-$C_7$-Alkyl substituiertes Carbamoyl steht und $R_3$ Wasserstoff oder $C_1$-$C_7$-Alkyl darstellt, mit der Massgabe, dass in Verbindungen der Formel I, in denen $R_1$ und $R_2$ gleich sind und jeweils für Carbamoyl oder N-Methylcarbamoyl stehen, mindestens ein Substituent von Ph in einer o-Stellung gebunden ist, wenn $R_3$ Wasserstoff darstellt, und mit der weiteren Massgabe, dass in Verbindungen der Formel I, worin Ph-alk Monohalogenphenyl-$C_1$-$C_2$-alkyl ist, $R_3$ für Wasserstoff steht, wenn einer der Reste $R_1$ und $R_2$ Carbamoyl ist und der andere Wasserstoff ist.

**2.** Verbindungen gemäss Anspruch 1, worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ Carbamoyl, N-$C_1$-$C_7$-Alkylcarbamoyl oder N,N-Di-$C_1$-$C_4$-alkylcarbamoyl bedeutet und der andere Wasserstoff, $C_1$-$C_7$-Alkyl, Carbamoyl, N-$C_1$-$C_7$-Alkylcarbamoyl oder N,N-Di-$C_1$-$C_4$-alkylcarbamoyl darstellt und $R_3$ für Wasserstoff oder $C_1$-$C_7$-Alkyl steht.

**3.** Verbindungen gemäss Anspruch 1, worin Ph durch $C_1$-$C_4$-Alkyl monosubstituiertes oder durch Halogen mit einer Atomnummer bis und mit 35 oder Halogen mit einer Atomnummer bis und mit 35 sowie $C_1$-$C_4$-Alkyl disubstituiertes Phenyl bedeutet, alk $C_1$-$C_4$-Alk-1-yliden oder $C_3$-$C_4$-Alk-2-yliden darstellt, einer der Reste $R_1$ und $R_2$ Carbamoyl oder N-$C_1$-$C_4$-Alkyl- oder N,N-Di-$C_1$-$C_4$-Alkylcarbamoyl darstellt und der andere Wasserstoff, Carbamoyl oder N-$C_1$-$C_4$-Alkyl- oder N,N-Di-$C_1$-$C_4$-Alkylcarbamoyl bedeutet und $R_3$ für Wasserstoff oder $C_1$-$C_4$-Alkyl steht.

**4.** Verbindungen gemäss einem der Ansprüche 1-3, in denen mindestens einer der genannten Substituenten von Ph in o-Stellung gebunden ist.

**5.** Verbindungen gemäss Anspruch 1, worin Ph o-$C_1$-$C_4$-Alkylphenyl, o-Halogenphenyl oder 2,6-, ferner 2,5-Dihalogenphenyl bedeutet, wobei Halogen jeweils eine Atomnummer bis und mit 35 aufweisen kann, alk $C_1$-$C_4$-Alk-1-yliden darstellt, einer der Reste $R_1$ und $R_2$ Carbamoyl, N-$C_1$-$C_4$-Alkylcarbamoyl oder N,N-Di-$C_1$-$C_4$-Alkylcarbamoyl und der andere Wasserstoff, Carbamoyl, N-$C_1$-$C_4$-Alkylcarbamoyl oder N,N-Di-$C_1$-$C_4$-Alkylcarbamoyl bedeutet und $R_3$ für Wasserstoff steht.

**6.** Verbindungen gemäss einem der Ansprüche 1-5, in denen alk Methylen, $R_1$ Wasserstoff oder Carbamoyl und $R_2$ Carbamoyl darstellt.

**7.** Verbindungen gemäss einem der Ansprüche 1-5, in denen alk Methylen, $R_1$ Wasserstoff und $R_2$ Carbamoyl darstellt.

**8.** Verbindungen gemäss Anspruch 1, worin Ph o-$C_1$-$C_4$-Alkylphenyl, o-Halogenphenyl oder 2,6-Dihalogenphenyl bedeutet, wobei Halogen jeweils eine Atomnummer bis und mit 35 aufweisen kann, alk für Methylen steht, $R_1$ Wasserstoff bedeutet, $R_2$ Carbamoyl, N-$C_1$-$C_4$-Alkylcarbamoyl oder N,N-Di-$C_1$-$C_4$-Alkylcarbamoyl bedeutet und $R_3$ Wasserstoff darstellt.

**9.** Verbindungen gemäss Anspruch 1, worin Ph 2,6-Dihalogenphenyl bedeutet, wobei Halogen jeweils eine Atomnummer bis und mit 35 aufweisen kann, alk für Methylen steht, $R_1$ Carbamoyl, N-$C_1$-$C_4$-Alkylcarbamoyl oder N,N-Di-$C_1$-$C_4$-Alkylcarbamoyl bedeutet, $R_2$ Wasserstoff, $C_1$-$C_4$-Alkyl, Carbamoyl oder mit $R_1$ identisches N-$C_1$-$C_4$-Alkylcarbamoyl oder N,N-Di-$C_1$-$C_4$-Alkylcarbamoyl bedeutet und $R_3$ Wasserstoff darstellt.

**10.** Verbindungen gemäss Anspruch 1, worin Ph 2,6-Dihalogenphenyl bedeutet, wobei Halogen jeweils eine Atomnummer bis und mit 35 aufweisen kann, alk für Methylen steht und $R_3$ Wasserstoff darstellt und worin entweder $R_1$ Wasserstoff und $R_2$ Carbamoyl, N-$C_1$-$C_4$-Alkylcarbamoyl oder N,N-Di-$C_1$-$C_4$-Alkylcarbamoyl bedeutet oder $R_1$ für Carbamoyl oder N-$C_1$-$C_4$-Alkylcarbamoyl und $R_2$ für Wasserstoff steht oder $R_1$ und $R_2$ beide Carbamoyl darstellen.

**11.** Verbindungen gemäss Anspruch 1, worin Ph o-$C_1$-$C_4$-Alkylphenyl, o-Halogenphenyl oder 2,6-Dihalogenphenyl bedeutet, wobei Halogen jeweils eine Atomnummer bis und mit 35 aufweisen kann, alk Methylen darstellt, $R_1$ Wasserstoff bedeutet, $R_2$ für Carbamoyl steht und $R_3$ Wasserstoff darstellt.

**12.** 1-(2,6-Difluorbenzyl)imidazol-4-carboxamid gemäss Anspruch 1.

**13.** 1-(o-Chlorbenzyl)imidazol-4-carboxamid gemäss Anspruch 1.

**14.** 1-(2,6-Difluorbenzyl)imidazol-4-(N-methyl)carboxamid gemäss Anspruch 1.

**15.** 1-(o-Fluorbenzyl)imidazol-4-carboxamid gemäss Anspruch 1.

**16.** 1-(2,6-Difluorbenzyl)imidazol-4-(N,N-dimethyl)carboxamid gemäss Anspruch 1.

**17.** 1-(2,6-Difluorbenzyl)imidazol-5-carboxamid gemäss Anspruch 1.

**18.** 1-(2,6-Difluorbenzyl)imidazol-4,5-dicarboxamid, 1-(2,5-Difluorbenzyl)imidazol-5-carboxamid, 1-(2,6-Difluorbenzyl)-5-methyl-imidazol-4-carboxamid, 1-(2,6-Difluorbenzyl)-2-methyl-imidazol-4-carboxamid, 1-(o-Methylbenzyl)imidazol-4-carboxamid, 1-[1-(2,6-Difluorphenyl)äthyl]imidazol-4-carboxamid oder 1-(2,6-Difluorbenzyl)imidazol-4-(N-acetyl)carboxamid gemäss Anspruch 1.

**19.** 1-(2,6-Difluorbenzyl)imidazol-4-(N-äthyl)carboxamid oder 1-(2,6-Difluorbenzyl)imidazol-5-(N-methyl)-carboxamid gemäss Anspruch 1.

**20.** 1-(2,6-Difluorbenzyl)imidazol-4,5-di(N,N-dimethyl)carboxamid oder 1-[1-(2,6-Difluorphenyl)äthyl]-imidazol-5-carboxamid gemäss Anspruch 1.

**21.** Verbindungen der Formel

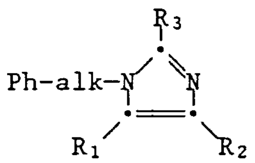

(I),

worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ gegebenenfalls ein- oder zweifach durch $C_1$-$C_7$-Alkyl substituiertes Carbamoyl und der andere Wasserstoff, $C_1$-$C_7$-Alkyl oder gegebenenfalls ein- oder zweifach durch $C_1$-$C_7$-Alkyl substituiertes Carbamoyl bedeutet und $R_3$ Wasserstoff oder $C_1$-$C_7$-Alkyl darstellt, mit der Massgabe, dass in Verbindungen der Formel I, worin Ph-alk Monohalogenphenyl-$C_1$-$C_2$-alkyl ist, $R_3$ für Wasserstoff steht, wenn einer der Reste $R_1$ und $R_2$ Carbamoyl ist und der andere Wasserstoff ist, zur Anwendung in einem Verfahren zur therapeutischen Behandlung des menschlichen oder tierischen Körpers.

**22.** Verbindungen gemäss einem der Ansprüche 1-6 und 11-18 zur Anwendung in einem Verfahren zur therapeutischen Behandlung des menschlichen oder tierischen Körpers.

**23.** Verbindungen gemäss einem der Ansprüche 8, 9 und 19 zur Anwendung in einem Verfahren zur therapeutischen Behandlung des menschlichen oder tierischen Körpers.

**24.** Verbindungen gemäss einem der Ansprüche 7, 10 und 20 zur Anwendung in einem Verfahren zur therapeutischen Behandlung des menschlichen oder tierischen Körpers.

**25.** Pharmazeutische Präparate enthaltend eine Verbindung gemäss einem der Ansprüche 1-6, 11-18, 21 und 22 neben üblichen pharmazeutischen Hilfsstoffen.

**26.** Pharmazeutische Präparate enthaltend eine Verbindung gemäss einem der Ansprüche 8, 9, 19 und 23 neben üblichen pharmazeutischen Hilfsstoffen.

**27.** Pharmazeutische Präparate enthaltend eine Verbindung gemäss einem der Ansprüche 7, 10, 20 und 24 neben üblichen pharmazeutischen Hilfsstoffen.

**28.** Verfahren zur Herstellung von Verbindungen der Formel

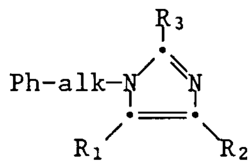 (I),

worin Ph durch $C_1$-$C_7$-Alkyl und/oder Kalogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ gegebenenfalls ein- oder zweifach durch $C_1$-$C_7$-Alkyl substituiertes Carbamoyl und der andere Wasserstoff, $C_1$-$C_7$-Alkyl oder gegebenenfalls ein- oder zweifach durch $C_1$-$C_7$-Alkyl substituiertes Carbamoyl bedeutet und $R_3$ Wasserstoff oder $C_1$-$C_7$-Alkyl darstellt, mit der Massgabe, dass in Verbindungen der Formel I, in denen $R_1$ und $R_2$ gleich sind und jeweils für Carbamoyl oder N-Methylcarbamoyl stehen, mindestens ein Substituent von Ph in einer o-Stellung gebunden ist, wenn $R_3$ Wasserstoff darstellt, und mit der weiteren Massgabe, dass in Verbindungen der Formel I, worin Ph-alk Monohalogenphenyl-$C_1$-$C_2$-alkyl ist, $R_3$ für Wasserstoff steht, wenn einer der Reste $R_1$ und $R_2$ Carbamoyl ist und der andere Wasserstoff ist, dadurch gekennzeichnet, dass man

a) in eine Verbindung der Formel

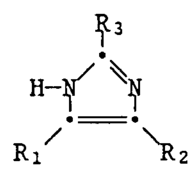 (II)

oder ein Salz davon den Rest der Formel Ph-alk- einführt oder

b) in einer Verbindung der Formel

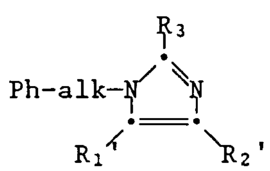 (III),

worin $R_1$' einen Rest $R_1$ oder X und $R_2$' einen Rest X oder $R_1$' einen Rest X und $R_2$' einen Rest $R_2$ darstellt, wobei X eine in gegebenenfalls ein- oder zweifach durch $C_1$-$C_7$-Alkyl substituiertes Carbamoyl überführbare Gruppe bedeutet, den Rest X bzw. die Reste X in gegebenenfalls ein- oder zweifach durch $C_1$-$C_7$-Alkyl substituiertes Carbamoyl überführt oder

c) zur Herstellung von Verbindungen der Formel I, worin $R_3$ Wasserstoff und $R_2$ di-$C_1$-$C_4$-alkyliertes Carbamoyl darstellt, eine Verbindung der Formel

Ph-alk-$NH_2$ (IV)

mit einem Isonitril der Formel

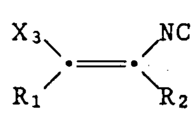 (V),

worin $X_3$ eine nukleofuge Abgangsgruppe darstellt, kondensiert und gewünschtenfalls jeweils ein gegebenenfalls erhaltenes Isomerengemisch in die Komponenten auftrennt und das gewünschte Isomere der Formel I isoliert, eine verfahrensgemäss erhältliche Verbindung der Formel I in eine andere Verbindung der Formel I überführt und/oder ein verfahrensgemass erhältliches

Diastereomeren- oder Enantiomerengemisch in die Diastereomeren oder Enantiomeren auftrennt.

**29.** Verwendung einer Verbindung gemäss einem der Ansprüche 1-24 zur Herstellung von zur Behandlung von Epilepsie bestimmten pharmazeutischen Präparaten.

**Patentansprüche für folgende Vertragsstaaten : AT, ES, GR**

**1.** Verfahren zur Herstellung von Verbindungen der Formel

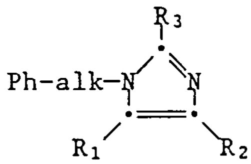

(I),

worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ gegebenenfalls ein- oder zweifach durch $C_1$-$C_7$-Alkyl substituiertes Carbamoyl und der andere Wasserstoff, $C_1$-$C_7$-Alkyl oder gegebenenfalls ein- oder zweifach durch $C_1$-$C_7$-Alkyl substituiertes Carbamoyl bedeutet und $R_3$ Wasserstoff oder $C_1$-$C_7$-Alkyl darstellt, mit der Massgabe, dass in Verbindungen der Formel I, in denen $R_1$ und $R_2$ gleich sind und jeweils für Carbamoyl oder N-Methylcarbamoyl stehen, mindestens ein Substituent von Ph in einer o-Stellung gebunden ist, wenn $R_3$ Wasserstoff darstellt, und mit der weiteren Massgabe, dass in Verbindungen der Formel I, worin Ph-alk Monohalogenphenyl-$C_1$-$C_2$-alkyl ist, $R_3$ für Wasserstoff steht, wenn einer der Reste $R_1$ und $R_2$ Carbamoyl ist und der andere Wasserstoff ist, dadurch gekennzeichnet, dass man

a) in eine Verbindung der Formel

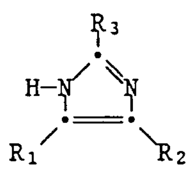

(II)

oder ein Salz davon den Rest der Formel Ph-alk- einführt oder

b) in einer Verbindung der Formel

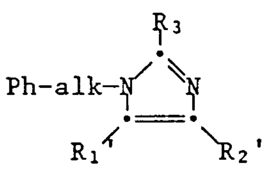

(III),

worin $R_1$' einen Rest $R_1$ oder X und $R_2$' einen Rest X oder $R_1$' einen Rest X und $R_2$' einen Rest $R_2$ darstellt, wobei X eine in gegebenenfalls ein- oder zweifach durch $C_1$-$C_7$-Alkyl substituiertes Carbamoyl überführbare Gruppe bedeutet, den Rest X bzw. die Reste X in gegebenenfalls ein- oder zweifach durch $C_1$-$C_7$-Alkyl substituiertes Carbamoyl überführt oder

c) zur Herstellung von Verbindungen der Formel I, worin $R_3$ Wasserstoff und $R_2$ di-$C_1$-$C_4$-alkyliertes Carbamoyl darstellt, eine Verbindung der Formel

$Ph\text{-}alk\text{-}NH_2$ (IV)

mit einem Isonitril der Formel

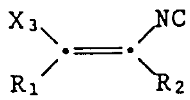

(V),

worin $X_3$ eine nukleofuge Abgangsgruppe darstellt, kondensiert und gewünschtenfalls jeweils ein gegebenenfalls erhaltenes Isomerengemisch in die Komponenten auftrennt und das gewünschte Isomere der Formel I isoliert, eine verfahrensgemäss erhältliche Verbindung der Formel I in eine andere Verbindung der Formel I überführt und/oder ein verfahrensgemäss erhältliches Diastereomeren- oder Enantiomerengemisch in die Diastereomeren oder Enantiomeren auftrennt.

**2.** Verfahren gemäss Anspruch 1, dadurch gekennzeichnet, dass man Verbindungen der Formel I, worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ Carbamoyl, N-$C_1$-$C_7$-Alkylcarbamoyl oder N,N-Di-$C_1$-$C_4$-alkylcarbamoyl bedeutet und der andere Wasserstoff, $C_1$-$C_7$-Alkyl, Carbamoyl, N-$C_1$-$C_7$-Alkylcarbamoyl oder N,N-Di-$C_1$-$C_4$-alkylcarbamoyl darstellt und $R_3$ für Wasserstoff oder $C_1$-$C_7$-Alkyl steht, herstellt.

**3.** Verfahren gemäss Anspruch 1, dadurch gekennzeichnet, dass man Verbindungen der-Formel I, worin Ph durch $C_1$-$C_4$-Alkylmonosubstituiertes oder durch Halogen mit einer Atomnummer bis und mit 35 oder Halogen mit einer Atomnummer bis und mit 35 sowie $C_1$-$C_4$-Alkyl disubstituiertes Phenyl bedeutet, alk $C_1$-$C_4$-Alk-1-yliden oder $C_3$-$C_4$-Alk-2-yliden darstellt, einer der Reste $R_1$ und $R_2$ Carbamoyl oder N-$C_1$-$C_4$-Alkyl- oder N,N-Di-$C_1$-$C_4$-Alkylcarbamoyl darstellt und der andere Wasserstoff, Carbamoyl oder N-$C_1$-$C_4$-Alkyl- oder N,N-Di-$C_1$-$C_4$-Alkylcarbamoyl bedeutet und $R_3$ für Wasserstoff oder $C_1$-$C_4$-Alkyl steht, herstellt.

**4.** Verfahren gemäss einem der Ansprüche 1-3, dadurch gekennzeichnet, dass man Verbindungen der Formel I, in denen mindestens einer der genannten Substituenten von Ph in o-Stellung gebunden ist, herstellt.

**5.** Verfahren gemäss Anspruch 1, dadurch gekennzeichnet, dass man Verbindungen der Formel I, worin Ph o-$C_1$-$C_4$-Alkylphenyl, o-Kalogenphenyl oder 2,6-, ferner 2,5-Dihalogenphenyl bedeutet, wobei Halogen jeweils eine Atomnummer bis und mit 35 aufweisen kann, alk $C_1$-$C_4$-Alk-1-yliden darstellt, einer der Reste $R_1$ und $R_2$ Carbamoyl, N-$C_1$-$C_4$-Alkylcarbamoyl oder N,N-Di-$C_1$-$C_4$-Alkylcarbamoyl und der andere Wasserstoff, Carbamoyl, N-$C_1$-$C_4$-Alkylcarbamoyl oder N,N-Di-$C_1$-$C_4$-Alkylcarbamoyl bedeutet und $R_3$ für Wasserstoff steht, herstellt.

**6.** Verfahren gemäss einem der Ansprüche 1-5, dadurch gekennzeichnet, dass man Verbindungen der Formel I, in denen alk Methylen, $R_1$ Wasserstoff oder Carbamoyl und $R_2$ Carbamoyl darstellt, herstellt.

**7.** Verfahren gemäss einem der Ansprüche 1-5, dadurch gekennzeichnet, dass man Verbindungen der Formel I, in denen alk Methylen, $R_1$ Wasserstoff und $R_2$ Carbamoyl darstellt, herstellt.

**8.** Verfahren gemäss Anspruch 1, dadurch gekennzeichnet, dass man Verbindungen der Formel I, worin Ph o-$C_1$-$C_4$-Alkylphenyl, o-Halogenphenyl oder 2,6-Dihalogenphenyl bedeutet, wobei Halogen jeweils eine Atomnummer bis und mit 35 aufweisen kann, alk für Methylen steht, $R_1$ Wasserstoff bedeutet, $R_2$ Carbamoyl, N-$C_1$-$C_4$-Alkylcarbamoyl oder N,N-Di-$C_1$-$C_4$-Alkylcarbamoyl bedeutet und $R_3$ Wasserstoff darstellt, herstellt.

**9.** Verfahren gemäss Anspruch 1, dadurch gekennzeichnet, dass man Verbindungen der Formel I, worin Ph 2,6-Dihalogenphenyl bedeutet, wobei Halogen jeweils eine Atomnummer bis und mit 35 aufweisen kann, alk für Methylen steht, $R_1$ Carbamoyl, N-$C_1$-$C_4$-Alkylcarbamoyl oder N,N-Di-$C_1$-$C_4$-Alkylcarbamoyl bedeutet, $R_2$ Wasserstoff, $C_1$-$C_4$-Alkyl, Carbamoyl oder mit $R_1$ identisches N-$C_1$-$C_4$-Alkylcarbamoyl oder N,N-Di-$C_1$-$C_4$-Alkylcarbamoyl bedeutet und $R_3$ Wasserstoff darstellt, herstellt.

**10.** Verfahren gemäss Anspruch 1, dadurch gekennzeichnet, dass man Verbindungen der Formel I, worin Ph 2,6-Dihalogenphenyl bedeutet, wobei Halogen jeweils eine Atomnummer bis und mit 35 aufweisen

kann, alk für Methylen steht und $R_3$ Wasserstoff darstellt und worin entweder $R_1$ Wasserstoff und $R_2$ Carbamoyl, N-$C_1$-$C_4$-Alkylcarbamoyl oder N,N-Di-$C_1$-$C_4$-Alkylcarbamoyl bedeutet oder $R_1$ für Carbamoyl oder N-$C_1$-$C_4$-Alkylcarbamoyl und $R_2$ für Wasserstoff steht oder $R_1$ und $R_2$ beide Carbamoyl darstellen, herstellt.

**11.** Verfahren gemäss Anspruch 1, dadurch gekennzeichnet, dass man Verbindungen der Formel I, worin Ph o-$C_1$-$C_4$-Alkylphenyl, o-Halogenphenyl oder 2,6-Dihalogenphenyl bedeutet, wobei Halogen jeweils eine Atomnummer bis und mit 35 aufweisen kann, alk Methylen darstellt, $R_1$ Wasserstoff bedeutet, $R_2$ für Carbamoyl steht und $R_3$ Wasserstoff darstellt, herstellt.

**12.** Verfahren gemäss Anspruch 1 zur Herstellung von 1-(2,6-Difluorbenzyl)imidazol-4-carboxamid.

**13.** Verfahren gemäss Anspruch 1 zur Herstellung von 1-(o-Chlorbenzyl)imidazol-4-carboxamid.

**14.** Verfahren gemäss Anspruch 1 zur Herstellung von 1-(2,6-Difluorbenzyl)imidazol-4-(N-methyl)-carboxamid.

**15.** Verfahren gemäss Anspruch 1 zur Herstellung von 1-(o-Fluorbenzyl)imidazol-4-carboxamid.

**16.** Verfahren gemäss Anspruch 1 zur Herstellung von 1-(2,6-Difluorbenzyl)imidazol-4-(N,N-dimethyl)-carboxamid.

**17.** Verfahren gemäss Anspruch 1 zur Herstellung von 1-(2,6-Difluorbenzyl)imidazol-5-carboxamid.

**18.** Verfahren gemäss Anspruch 1 zur Herstellung von 1-(2,6-Difluorbenzyl)imidazol-4,5-dicarboxamid, 1-(2,5-Difluorbenzyl)imidazol-5-carboxamid, 1-(2,6-Difluorbenzyl)-5-methyl-imidazol-4-carboxamid, 1-(2,6-Difluorbenzyl)-2-methyl-imidazol-4-carboxamid, 1-(o-Methylbenzyl)imidazol-4-carboxamid, 1-[1-(2,6-Difluorphenyl)äthyl]imidazol-4-carboxamid, 1-(2,6-Difluorbenzyl)imidazol-4-(N-acetyl)carboxamid, 1-(2,6-Difluorbenzyl)imidazol-4-(N-äthyl)carboxamid, 1-(2,6-Difluorbenzyl)imidazol-5-(N-methyl)carboxamid, 1-(2,6-Difluorbenzyl)imidazol-4,5-di(N,N-dimethyl)carboxamid oder 1-[1-(2,6-Difluorphenyl)äthyl]imidazol-5-carboxamid.

**19.** Verfahren zur Herstellung pharmazeutischer Präparate, dadurch gekennzeichnet, dass man eine Verbindung der Formel

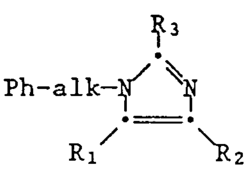

(I),

worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ gegebenenfalls

ein- oder zweifach durch $C_1$-$C_7$-Alkyl substituiertes Carbamoyl und der andere Wasserstoff, $C_1$-$C_7$-Alkyl oder gegebenenfalls ein- oder zweifach durch $C_1$-$C_7$-Alkyl substituiertes Carbamoyl bedeutet und $R_3$ Wasserstoff oder $C_1$-$C_7$-Alkyl darstellt, mit der Massgabe, dass in Verbindungen der Formel I, worin Ph-alk Monohalogenphenyl-$C_1$-$C_2$-alkyl ist, $R_3$ für Wasserstoff steht, wenn einer der Reste $R_1$ und $R_2$ Carbamoyl ist und der andere Wasserstoff ist, mit üblichen pharmazeutischen Hilfsstoffen vermischt.

**20.** Verfahren zur Herstellung pharmazeutischer Präparate, dadurch gekennzeichnet, dass man

a) in eine Verbindung der Formel

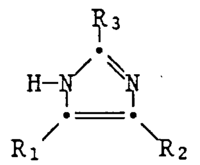

(II)

oder ein Salz davon den Rest der Formel Ph-alk- einführt oder

b) in einer Verbindung der Formel

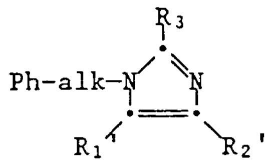

(III),

worin $R_1$' einen Rest $R_1$ oder X und $R_2$' einen Rest X oder $R_1$' einen Rest X und $R_2$' einen Rest $R_2$ darstellt, wobei X eine in gegebenenfalls ein- oder zweifach durch $C_1$-$C_7$-Alkyl substituiertes Carbamoyl überführbare Gruppe bedeutet, den Rest X bzw. die Reste X in gegebenenfalls ein- oder zweifach durch $C_1$-$C_7$-Alkyl substituiertes Carbamoyl überführt oder

c) zur Herstellung von Verbindungen der Formel I, worin $R_3$ Wasserstoff und $R_2$ di-$C_1$-$C_4$-alkyliertes Carbamoyl darstellt, eine Verbindung der Formel

$Ph\text{-}alk\text{-}NH_2$ (IV)

mit einem Isonitril der Formel

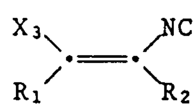

(V),

worin $X_3$ eine nukleofuge Abgangsgruppe darstellt, kondensiert und gewünschtenfalls jeweils ein gegebenenfalls erhaltenes Isomerengemisch in die Komponenten auftrennt und das gewünschte Isomere der Formel I isoliert, eine verfahrensgemäss erhältliche Verbindung der Formel I in eine andere Verbindung der Formel I überführt und/oder ein verfahrensgemäss erhältliches Diastereomeren- oder Enantiomerengemisch in die Diastereomeren oder Enantiomeren auftrennt und die erhaltene Verbindung der Formel

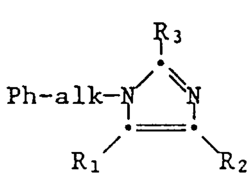

(I),

worin Ph durch $C_1$-$C_7$-Alkyl und/oder Halogen mit einer Atomnummer bis und mit 35 mono-, di- oder trisubstituiertes Phenyl bedeutet, alk $C_1$-$C_4$-Alkyliden darstellt, einer der Reste $R_1$ und $R_2$ gegebenenfalls ein- oder zweifach durch $C_1$-$C_7$-Alkyl substituiertes Carbamoyl und der andere Wasserstoff, $C_1$-$C_7$-Alkyl oder gegebenenfalls ein- oder zweifach durch $C_1$-$C_7$-Alkyl substituiertes Carbamoyl bedeutet und $R_3$ Wasserstoff oder $C_1$-$C_7$-Alkyl darstellt, mit der Massgabe, dass in Verbindungen der Formel I, worin Ph-alk Monohalogenphenyl-$C_1$-$C_2$-alkyl ist, $R_3$ für Wasserstoff steht, wenn einer der Reste $R_1$ und $R_2$ Carbamoyl ist und der andere Wasserstoff ist, mit üblichen pharmazeutischen Hilfsstoffen vermischt.

## Claims

**Claims for the following Contracting States : BE, CH, DE, FR, GB, IT, LI, LU, NL, SE**

1. A compound of the formula

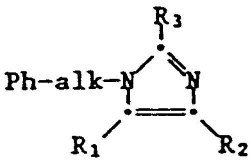

(I)

in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl optionally mono- or di-substituted by $C_1$-$C_7$alkyl and the other is hydrogen, $C_1$-$C_7$alkyl, or carbamoyl optionally mono- or di-substituted by $C_1$-$C_7$alkyl, and $R_3$ represents hydrogen or $C_1$-$C_7$alkyl, with the proviso that, in compounds of the formula I in which $R_1$ and $R_2$ are the same and each represents carbamoyl or N-methylcarbamoyl, if $R_3$ represents hydrogen at least one substituent of Ph is bonded in the o-position, and with the further proviso that, in compounds of the formula I in which Ph-alk is monohalophenyl-$C_1$-$C_2$alkyl, if one of the radicals $R_1$ and $R_2$ is carbamoyl and the other is hydrogen, $R_3$ is hydrogen.

2. A compound according to claim 1, in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl, N-$C_1$-$C_7$alkylcarbamoyl or N,N-di-$C_1$-$C_4$alkylcarbamoyl and the other is hydrogen, $C_1$-$C_7$alkyl, carbamoyl, N-$C_1$-$C_7$alkylcarbamoyl or N,N-di-$C_1$-$C_4$alkylcarbamoyl, and $R_3$ represents hydrogen or $C_1$-$C_7$alkyl.

3. A compound according to claim 1, in which Ph represents phenyl that is monosubstituted by $C_1$-$C_4$alkyl or disubstituted by halogen having an atomic number of up to and including 35 or by halogen having an atomic number of up to and including 35 and $C_1$-$C_4$alkyl, alk represents $C_1$-$C_4$alk-1-ylidene or $C_3$-$C_4$alk-2-ylidene, one of the radicals $R_1$ and $R_2$ is carbamoyl or N-$C_1$-$C_4$alkyl- or N,N-di-$C_1$-$C_4$alkyl-carbamoyl and the other is hydrogen, carbamoyl or N-$C_1$-$C_4$alkyl- or N,N-di-$C_1$-$C_4$alkylcarbamoyl, and $R_3$ represents hydrogen or $C_1$-$C_4$alkyl.

4. A compound according to any one of claims 1 to 3, in which at least one of the mentioned substituents of Ph is bonded in the o-position.

5. A compound according to claim 1, in which Ph represents o-$C_1$-$C_4$alkylphenyl, o-halophenyl or 2,6- or 2,5-dihalophenyl, where halogen may in each case have an atomic number of up to and including 35, alk represents $C_1$-$C_4$alk-1-ylidene, one of the radicals $R_1$ and $R_2$ is carbamoyl, N-$C_1$-$C_4$alkylcarbamoyl or N,N-di-$C_1$-$C_4$alkylcarbamoyl and the other is hydrogen, carbamoyl, N-$C_1$-$C_4$alkylcarbamoyl or N,N-di-$C_1$-$C_4$alkylcarbamoyl, and $R_3$ represents hydrogen.

6. A compound according to any one of claims 1 to 5, in which alk represents methylene, $R_1$ represents hydrogen or carbamoyl and $R_2$ represents carbamoyl.

7. A compound according to any one of claims 1 to 5, in which alk represents methylene, $R_1$ represents hydrogen and $R_2$ represents carbamoyl.

8. A compound according to claim 1, in which Ph represents o-$C_1$-$C_4$alkylphenyl, o-halophenyl or 2,6-dihalophenyl, where halogen may in each case have an atomic number of up to and including 35, alk represents methylene, $R_1$ represents hydrogen, $R_2$ represents carbamoyl, N-$C_1$-$C_4$alkylcarbamoyl or N,N-di-$C_1$-$C_4$alkylcarbamoyl, and $R_3$ represents hydrogen.

9. A compound according to claim 1, in which Ph represents 2,6-dihalophenyl, where halogen may in each case have an atomic number of up to and including 35, alk represents methylene, $R_1$ represents carbamoyl, N-$C_1$-$C_4$alkylcarbamoyl or N,N-di-$C_1$-$C_4$alkylcarbamoyl, $R_2$ represents hydrogen, $C_1$-$C_4$alkyl or carbamoyl, or represents N-$C_1$-$C_4$alkylcarbamoyl or N,N-di-$C_1$-$C_4$alkylcarbamoyl, each identical to

$R_1$, and $R_3$ represents hydrogen.

**10.** A compound according to claim 1, in which Ph represents 2,6-dihalophenyl, where halogen may in each case have an atomic number of up to and including 35, alk represents methylene and $R_3$ represents hydrogen, and in which either $R_1$ represents hydrogen and $R_2$ represents carbamoyl, N-$C_1$-$C_4$alkylcarbamoyl or N,N-di-$C_1$-$C_4$alkylcarbamoyl, or $R_1$ represents carbamoyl or N-$C_1$-$C_4$alkylcarbamoyl and $R_2$ represents hydrogen, or $R_1$ and $R_2$ both represent carbamoyl.

**11.** A compound according to claim 1, in which Ph represents o-$C_1$-$C_4$alkylphenyl, o-halophenyl or 2,6-dihalophenyl, where halogen may in each case have an atomic number of up to and including 35, alk represents methylene, $R_1$ represents hydrogen, $R_2$ represents carbamoyl and $R_3$ represents hydrogen.

**12.** 1-(2,6-Difluorobenzyl)imidazole-4-carboxamide according to claim 1.

**13.** 1-(o-Chlorobenzyl)imidazole-4-carboxamide according to claim 1.

**14.** 1-(2,6-Difluorobenzyl)imidazole-4-(N-methyl)carboxamide according to claim 1.

**15.** 1-(o-Fluorobenzyl)imidazole-4-carboxamide according to claim 1.

**16.** 1-(2,6-Difluorobenzyl)imidazole-4-(N,N-dimethyl)carboxamide according to claim 1.

**17.** 1-(2,6-Difluorobenzyl)imidazole-5-carboxamide according to claim 1.

**18.** 1-(2,6-Difluorobenzyl)imidazole-4,5-dicarboxamide, 1-(2,5-difluorobenzyl)imidazole-5-carboxamide, 1-(2,6-dirluorobenzyl)-5-methylimidazole-4-carboxamide, 1-(2,6-difluorobenzyl)-2-methylimidazole-4-carboxamide, 1-(o-methylbenzyl)imidazole-4-carboxamide, 1-[1-(2,6-difluorophenyl)ethyl]imidazole-4-carboxamide or 1-(2,6-difluorobenzyl)imidazole-4-(N-acetyl)carboxamide according to claim 1.

**19.** 1-(2,6-Difluorobenzyl)imidazole-4-(N-ethyl)carboxamide or 1-(2,6-difluorobenzyl)imidazole-5-(N-methyl)-carboxamide according to claim 1.

**20.** 1-(2,6-Difluorobenzyl)imidazole-4,5-di(N,N-dimethyl)carboxamide or 1-[1-(2,6-difluorophenyl)ethyl]-imidazole-5-carboxamide according to claim 1.

**21.** A compound of the formula

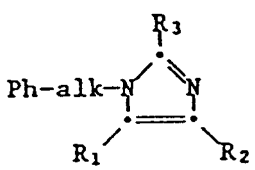

(I)

in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl optionally mono- or di-substituted by $C_1$-$C_7$alkyl and the other is hydrogen, $C_1$-$C_7$alkyl, or carbamoyl optionally mono- or di-substituted by $C_1$-$C_7$alkyl, and $R_3$ represents hydrogen or $C_1$-$C_7$alkyl, with the proviso that, in compounds of the formula I in which Ph-alk is mono-halophenyl-$C_1$-$C_2$alkyl, if one of the radicals $R_1$ and $R_2$ is carbamoyl and the other is hydrogen, $R_3$ is hydrogen, for use in a method for the therapeutic treatment of the human or animal body.

**22.** A compound according to any one of claims 1 to 6 and 11 to 18 for use in a method for the therapeutic treatment of the human or animal body.

**23.** A compound according to any one of claims 8, 9 and 19 for use in a method for the therapeutic treatment of the human or animal body.

**24.** A compound according to any one of claims 7, 10 and 20 for use in a method for the therapeutic treatment of the human or animal body.

**25.** A pharmaceutical preparation comprising a compound according to any one of claims 1 to 6, 11 to 18, 21 and 22, in addition to customary pharmaceutical adjuncts.

**26.** A pharmaceutical preparation comprising a compound according to any one of claims 8, 9, 19 and 23, in addition to customary pharmaceutical adjuncts.

**27.** A pharmaceutical preparation comprising a compound according to any one of claims 7, 10, 20 and 24, in addition to customary pharmaceutical adjuncts.

**28.** A process for the manufacture of a compound of the formula

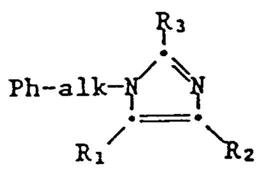

(I)

in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl optionally mono- or di-substituted by $C_1$-$C_7$alkyl and the other is hydrogen, $C_1$-$C_7$alkyl, or carbamoyl optionally mono- or di-substituted by $C_1$-$C_7$alkyl, and $R_3$ represents hydrogen or $C_1$-$C_7$alkyl, with the proviso that, in compounds of the formula I in which $R_1$ and $R_2$ are the same and each represents carbamoyl or N-methylcarbamoyl, if $R_3$ represents hydrogen at least one substituent of Ph is bonded in the o-position, and with the further proviso that, in compounds of the formula I in which Ph-alk is monohalophenyl-$C_1$-$C_2$alkyl, if one of the radicals $R_1$ and $R_2$ is carbamoyl and the other is hydrogen, $R_3$ is hydrogen, which process comprises

a) introducing the radical of the formula Ph-alk- into a compound of the formula

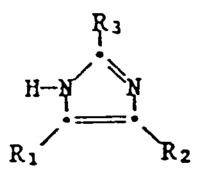

(II)

or into a salt thereof, or

b) in a compound of the formula

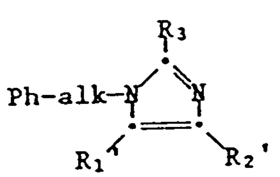

(III)

in which $R_1$' represents a radical $R_1$ or X and $R_2$' represents a radical X, or $R_1$' represents a radical X and $R_2$' represents a radical $R_2$, where X represents a group that can be converted into carbamoyl that is optionally mono- or di-substituted by $C_1$-$C_7$alkyl, converting the radical X or the radicals X into carbamoyl that is optionally mono- or di-substituted by $C_1$-$C_7$alkyl, or

c) for the manufacture of a compound of the formula I in which $R_3$ represents hydrogen and $R_2$ represents di-$C_1$-$C_4$alkylated carbamoyl$_1$ condensing a compound of the formula

Ph-alk-$NH_2$ (IV)

with an isonitrile of the formula

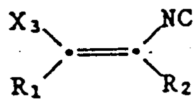 (V)

in which $X_3$ represents a nucleofugal leaving group, and if desired, in each case, separating an isomeric mixture which may be obtained into its components and isolating the desired isomer of the formula I, converting a compound of the formula I obtainable in accordance with the process into a different compound of the formula I and/or separating a diastereoisomeric or enantiomeric mixture obtainable in accordance with the process into the diastereoisomers or enantiomers.

**29.** The use of a compound according to any one of claims 1 to 24 for the manufacture of a pharmaceutical preparation for the treatment of epilepsy.

**Claims for the following Contracting States : AT, ES, GR**

**1.** A process for the manufacture of a compound of the formula

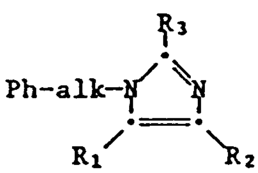 (I)

in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl optionally mono- or di-substituted by $C_1$-$C_7$alkyl and the other is hydrogen, $C_1$-$C_7$alkyl, or carbamoyl optionally mono- or di-substituted by $C_1$-$C_7$alkyl, and $R_3$ represents hydrogen or $C_1$-$C_7$alkyl, with the proviso that, in compounds of the formula I in which $R_1$ and $R_2$ are the same and each represents carbamoyl or N-methylcarbamoyl, if $R_3$ represents hydrogen at least one substituent of Ph is bonded in the o-position, and with the further proviso that, in compounds of the formula I in which Ph-alk is monohalophenyl-$C_1$-$C_2$alkyl, if one of the radicals $R_1$ and $R_2$ is carbamoyl and the other is hydrogen, $R_3$ is hydrogen, which process comprises

a) introducing the radical of the formula Ph-alk- into a compound of the formula

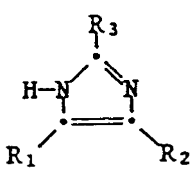 (II)

or into a salt thereof, or

b) in a compound of the formula

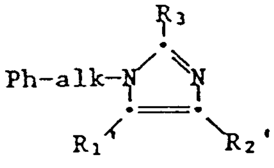 (III)

in which $R_1'$ represents a radical $R_1$ or X and $R_2'$ represents a radical X, or $R_1'$ represents a radical X and $R_2'$ represents a radical $R_2$, where X represents a group that can be converted into carbamoyl that is optionally mono- or di-substituted by $C_1$-$C_7$alkyl, converting the radical X or the radicals X into carbamoyl that is optionally mono- or di-substituted by $C_1$-$C_7$alkyl, or
c) for the manufacture of a compound of the formula I in which $R_3$ represents hydrogen and $R_2$ represents di-$C_1$-$C_4$alkylated carbamoyl, condensing a compound of the formula

Ph-alk-$NH_2$ (IV)

with an isonitrile of the formula

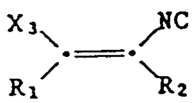

(V)

in which $X_3$ represents a nucleofugal leaving group, and if desired, in each case, separating an isomeric mixture which may be obtained into its components and isolating the desired isomer of the formula I, converting a compound of the formula I obtainable in accordance with the process into a different compound of the formula I and/or separating a diastereoisomeric or enantiomeric mixture obtainable in accordance with the process into the diastereoisomers or enantiomers.

**2.** A process according to claim 1, wherein there is manufactured a compound of the formula I in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl, N-$C_1$-$C_7$alkylcarbamoyl or N,N-di-$C_1$-$C_4$alkylcarbamoyl and the other is hydrogen, $C_1$-$C_7$alkyl, carbamoyl, N-$C_1$-$C_7$alkylcarbamoyl or N,N-di-$C_1$-$C_4$alkylcarbamoyl, and $R_3$ represents hydrogen or $C_1$-$C_7$alkyl.

**3.** A process according to claim 1, wherein there is manufactured a compound of the formula I in which Ph represents phenyl that is monosubstituted by $C_1$-$C_4$alkyl or disubstituted by halogen having an atomic number of up to and including 35 or by halogen having an atomic number of up to and including 35 and $C_1$-$C_4$alkyl, alk represents $C_1$-$C_4$alk-1-ylidene or $C_3$-$C_4$alk-2-ylidene, one of the radicals $R_1$ and $R_2$ is carbamoyl or N-$C_1$-$C_4$alkyl- or N,N-di-$C_1$-$C_4$alkyl-carbamoyl and the other is hydrogen, carbamoyl or N-$C_1$-$C_4$alkyl- or N,N-di-$C_1$-$C_4$alkylcarbamoyl, and $R_3$ represents hydrogen or $C_1$-$C_4$alkyl.

**4.** A process according to any one of claims 1 to 3, wherein there is manufactured a compound of the formula I in which at least one of the mentioned substituents of Ph is bonded in the o-position.

**5.** A process according to claim 1, wherein there is manufactured a compound of the formula I in which Ph represents o-$C_1$-$C_4$alkylphenyl, o-halophenyl or 2,6- or 2,5-dihalophenyl, where halogen may in each case have an atomic number of up to and including 35, alk represents $C_1$-$C_4$alk-1-ylidene, one of the radicals $R_1$ and $R_2$ is carbamoyl, N-$C_1$-$C_4$alkylcarbamoyl or N,N-di-$C_1$-$C_4$alkylcarbamoyl and the other is hydrogen, carbamoyl, N-$C_1$-$C_4$alkylcarbamoyl or N,N-di-$C_1$-$C_4$alkylcarbamoyl, and $R_3$ represents hydrogen.

**6.** A process according to any one of claims 1 to 5, wherein there is manufactured a compound of the formula I in which alk represents methylene, $R_1$ represents hydrogen or carbamoyl and $R_2$ represents carbamoyl.

**7.** A process according to any one of claims 1 to 5, wherein there is manufactured a compound of the formula I in which alk represents methylene, $R_1$ represents hydrogen and $R_2$ represents carbamoyl.

**8.** A process according to claim 1, wherein there is manufactured a compound of the formula I in which Ph represents o-$C_1$-$C_4$alkylphenyl, o-halophenyl or 2,6-dihalophenyl, where halogen may in each case have an atomic number of up to and including 35, alk represents methylene, $R_1$ represents hydrogen, $R_2$ represents carbamoyl, N-$C_1$-$C_4$alkylcarbamoyl or N,N-di-$C_1$-$C_4$alkylcarbamoyl, and $R_3$ represents

hydrogen.

**9.** A process according to claim 1, wherein there is manufactured a compound of the formula I in which Ph represents 2,6-dihalophenyl, where halogen may in each case have an atomic number of up to and including 35, alk represents methylene, $R_1$ represents carbamoyl, N-$C_1$-$C_4$alkylcarbamoyl or N,N-di-$C_1$-$C_4$alkylcarbamoyl, $R_2$ represents hydrogen, $C_1$-$C_4$alkyl or carbamoyl, or represents N-$C_1$-$C_4$alkylcarbamoyl or N,N-di-$C_1$-$C_4$alkylcarbamoyl, each identical to $R_1$, and $R_3$ represents hydrogen.

**10.** A process according to claim 1, wherein there is manufactured a compound of the formula I in which Ph represents 2,6-dihalophenyl, where halogen may in each case have an atomic number of up to and including 35, alk represents methylene and $R_3$ represents hydrogen, and in which either $R_1$ represents hydrogen and $R_2$ represents carbamoyl, N-$C_1$-$C_4$alkylcarbamoyl or N,N-di-$C_1$-$C_4$alkylcarbamoyl, or $R_1$ represents carbamoyl or N-$C_1$-$C_4$alkylcarbamoyl and $R_2$ represents hydrogen, or $R_1$ and $R_2$ both represent carbamoyl.

**11.** A process according to claim 1, wherein there is manufactured a compound of the formula I in which Ph represents o-$C_1$-$C_4$alkylphenyl, o-halophenyl or 2,6-dihalophenyl, where halogen may in each case have an atomic number of up to and including 35, alk represents methylene, $R_1$ represents hydrogen, $R_2$ represents carbamoyl and $R_3$ represents hydrogen.

**12.** A process according to claim 1 for the manufacture of 1-(2,6-difluorobenzyl)imidazole-4-carboxamide.

**13.** A process according to claim 1 for the manufacture of 1-(ochlorobenzyl)imidazole-4-carboxamide.

**14.** A process according to claim 1 for the manufacture of 1-(2,6-difluorobenzyl)imidazole-4-(N-methyl)-carboxamide.

**15.** A process according to claim 1 for the manufacture of 1-(o-fluorobenzyl)imidazole-4-carboxamide.

**16.** A process according to claim 1 for the manufacture of 1-(2,6-difluorobenzyl)imidazole-4-(N,N-dimethyl)-carboxamide.

**17.** A process according to claim 1 for the manufacture of 1-(2,6-difluorobenzyl)imidazole-5-carboxamide.

**18.** A process according to claim 1 for the manufacture of 1-(2,6-difluorobenzyl)imidazole-4,5-dicarboxamide, 1-(2,5-difluorobenzyl)imidazole-5-carboxamide, 1-(2,6-difluorobenzyl)-5-methylimidazole-4-carboxamide, 1-(2,6-difluorobenzyl)-2-methylimidazole-4-carboxamide, 1-(o-methylbenzyl)imidazole-4-carboxamide, 1-[1-(2,6-difluorophenyl)ethyl]imidazole-4-carboxamide, 1-(2,6-difluorobenzyl)imidazole-4-(N-acetyl)carboxamide, 1-(2,6-difluorobenzyl)imidazole-4-(N-ethyl)carboxamide, 1-(2,6-difluorobenzyl)-imidazole-5-(N-methyl)carboxamide, 1-(2,6-difluorobenzyl)imidazole-4,5-di(N,N-dimethyl)carboxamide or 1-[1-(2,6-difluorophenyl)ethyl]imidazole-5-carboxamide.

**19.** A process for the manufacture of a pharmaceutical preparation, which comprises mixing with customary pharmaceutical adjuncts a compound of the formula

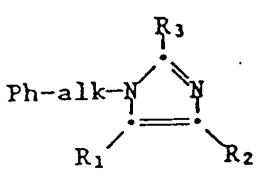

(I)

in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl optionally mono- or di-substituted by $C_1$-$C_7$-alkyl and the other is hydrogen, $C_1$-$C_7$alkyl, or carbamoyl optionally mono- or di-substituted by $C_1$-$C_7$alkyl, and $R_3$ represents hydrogen or $C_1$-$C_7$alkyl, with the proviso that, in compounds of the formula I in which Ph-alk is mono-halophenyl-

$C_1$-$C_2$alkyl, if one of the radicals $R_1$ and $R_2$ is carbamoyl and the other is hydrogen, $R_3$ is hydrogen.

**20.** A process for the manufacture of a pharmaceutical preparation, which comprises
a) introducing the radical of the formula Ph-alk- into a compound of the formula

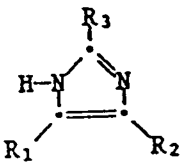

(II)

or into a salt thereof, or
b) in a compound of the formula

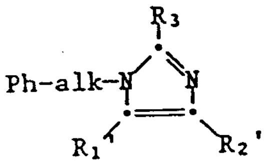

(III)

in which $R_1$' represents a radical $R_1$ or X and $R_2$' represents a radical X, or $R_1$' represents a radical X and $R_2$' represents a radical $R_2$, where X represents a group that can be converted into carbamoyl that is optionally mono- or di-substituted by $C_1$-$C_7$alkyl, converting the radical X or the radicals X into carbamoyl that is optionally mono- or di-substituted by $C_1$-$C_7$alkyl, or
c) for the manufacture of a compound of the formula I in which $R_3$ represents hydrogen and $R_2$ represents di-$C_1$-$C_4$alkylated carbamoyl, condensing a compound of the formula

Ph-alk-$NH_2$ (IV)

with an isonitrile of the formula

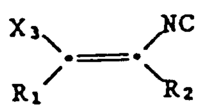

(V)

in which $X_3$ represents a nucleofugal leaving group, and if desired, in each case, separating an isomeric mixture which may be obtained into its components and isolating the desired isomer of the formula I, converting a compound of the formula I obtainable in accordance with the process into a different compound of the formula I and/or separating a diastereoisomeric or enantiomeric mixture obtainable in accordance with the process into the diastereoisomers or enantiomers, and mixing the resulting compound of the formula

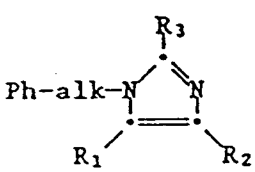

(I),

in which Ph represents phenyl that is mono-, di- or tri-substituted by $C_1$-$C_7$alkyl and/or by halogen having an atomic number of up to and including 35, alk represents $C_1$-$C_4$alkylidene, one of the radicals $R_1$ and $R_2$ is carbamoyl optionally mono- or di-substituted by $C_1$-$C_7$-alkyl and the other is hydrogen, $C_1$-$C_7$alkyl, or carbamoyl optionally mono- or di-substituted by $C_1$-$C_7$alkyl, and $R_3$

represents hydrogen or $C_1$-$C_7$alkyl, with the proviso that, in compounds of the formula I in which Ph-alk is mono-halophenyl-$C_1$-$C_2$alkyl, if one of the radicals $R_1$ and $R_2$ is carbamoyl and the other is hydrogen, $R_3$ is hydrogen, with customary pharmaceutical adjuncts.

## Revendications

**Revendications pour les Etats contractants suivants : BE, CH, DE, FR, GB, IT, LI, LU, NL, SE**

**1.** Composés de formule

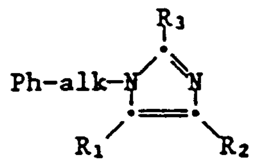

(I),

dans laquelle Ph représente un groupe phényle mono-, di-ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle éventuellement mono- ou di-substitué par des groupes alkyle en C 1-C 7 et l'autre l'hydrogène, un groupe alkyle en C 1-C 7 ou un groupe carbamoyle éventuellement mono- ou di-substitué par des groupes alkyle en C 1-C 7 et $R_3$ représente l'hydrogène ou un groupe alkyle en C 1-C 7, sous réserve que, dans les composés de formule I pour lesquels $R_1$ et $R_2$ sont identiques et représentent chacun un groupe carbamoyle ou N-méthylcarbamoyle, l'un au moins des substituants de Ph est relié en position ortho lorsque $R_3$ représente l'hydrogène, et sous réserve également que, dans les composés de formule I pour lesquels Ph-alk représente un groupe monohalogénophényl-alkyle en C 1-C 2, $R_3$ représente l'hydrogène lorsque l'un des symboles $R_1$ et $R_2$ représente un groupe carbamoyle et l'autre l'hydrogène.

**2.** Composés selon revendication 1, dans lesquels Ph représente un groupe phényle mono-, di- ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle, N-(alkyle en C 1-C 7)-carbamoyle ou N,N-di-(alkyle en C 1-C 4)-carbamoyle et l'autre l'hydrogène, un groupe alkyle en C 1-C 7, carbamoyle, N-(alkyle en C 1-C 7)-carbamoyle ou N,N-di-(alkyle en C 1-C 4)-carbamoyle et $R_3$ représente l'hydrogène ou un groupe alkyle en C 1-C 7.

**3.** Composés selon revendication 1, dans lesquels Ph représente un groupe phényle monosubstitué par un groupe alkyle en C 1-C 4 ou disubstitué par des halogènes de numéro atomique allant jusqu'à 35 inclus ou par un halogène de numéro atomique allant jusqu'à 35 inclus et un groupe alkyle en C 1-C 4, alk représente un groupe alca-1-ylidène en C 1-C 4 ou alca-2-ylidène en C 3-C 4, l'un des symboles $R_1$ et $R_2$ représente un groupe carbamoyle ou N-(alkyle en C 1-C 4)- ou N,N-di-(alkyle en C 1-C 4)-carbamoyle et l'autre l'hydrogène, un groupe carbamoyle ou N-(alkyle en C 1-C 4)- ou N,N-di-(alkyle en C 1-C 4)-carbamoyle et $R_3$ représente l'hydrogène ou un groupe alkyle en C 1-C 4.

**4.** Composés selon l'une des revendications 1 à 3, dans lesquels l'un au moins des substituants mentionnés pour Ph est relié en position ortho.

**5.** Composés selon la revendication 1, dans lesquels Ph représente un groupe o-(alkyle en C 1-C 4)-phényle, o-halogénophényle ou 2,6- ou encore 2,5-dihalogénophényle, les halogènes pouvant tous avoir un numéro atomique allant jusqu'à 35 inclus, alk représente un groupe alca-1-ylidène en C 1-C 4, l'un des symboles $R_1$ et $R_2$ représente un groupe carbamoyle, N-(alkyle en C 1-C 4)-carbamoyle ou N,N-di-(alkyle en C 1-C 4)-carbamoyle et l'autre l'hydrogène, un groupe carbamoyle, N-(alkyle en C 1-C 4)-carbamoyle ou N,N-di-(alkyle en C 1-C 4)-carbamoyle et $R_3$ représente l'hydrogène.

**6.** Composés selon l'une des revendications 1 à 5, dans lesquels alk représente un groupe méthylène, $R_1$ l'hydrogène ou un groupe carbamoyle et $R_2$ un groupe carbamoyle.

**7.** Composés selon l'une des revendications 1 à 5, dans lesquels alk représente un groupe méthylène, $R_1$ représente l'hydrogène et $R_2$ un groupe carbamoyle.

**8.** Composés selon la revendication 1, dans lesquels Ph représente un groupe o-(alkyle en C 1-C 4)-phényle, o-halogénophényle ou 2,6-dihalogénophényle, les halogènes en question pouvant tous avoir un numéro atomique allant jusqu'à 35 inclus, alk représente un groupe méthylène, $R_1$ l'hydrogène, $R_2$ un groupe carbamoyle, N-(alkyle en C 1-C 4)-carbamoyle ou N,N-di-(alkyle en C 1-C 4)-carbamoyle, et $R_3$ l'hydrogène.

**9.** Composés selon revendication 1, dans lesquels Ph représente un groupe 2,6-dihalogénophényle dont les halogènes peuvent tous deux avoir un numéro atomique allant jusqu'à 35 inclus, alk représente un groupe méthylène, $R_1$ un groupe carbamoyle, N-(alkyle en C 1-C 4)-carbamoyle ou N,N-di-(alkyle en C 1-C 4)-carbamoyle, $R_2$ représente l'hydrogène, un groupe alkyle en C 1-C 4, carbamoyle ou bien, comme $R_1$, un groupe N-(alkyle en C 1-C 4)-carbamoyle ou N,N-di-(alkyle en C 1-C 4)-carbamoyle et $R_3$ représente l'hydrogène.

**10.** Composés selon la revendication 1, dans lesquels Ph représente un groupe 2,6-dihalogénophényle dont les halogènes peuvent tous deux avoir un numéro atomique allant jusqu'à 35 inclus, alk représente un groupe méthylène et $R_3$ l'hydrogène, et dans lesquels ou bien $R_1$ représentel'hydrogène et $R_2$ un groupe carbamoyle, N-(alkyle en C 1-C 4)-carbamoyle ou N,N-di-(alkyle en C 1-C 4)-carbamoyle, ou bien $R_1$ représente un groupe carbamoyle ou N-(alkyle en C 1-C 4)-carbamoyle et $R_2$ l'hydrogène, ou Lien $R_1$ et $R_2$ représentent tous deux des groupes carbamoyle.

**11.** Composés selon la revendication 1, dans lesquels Ph représente un groupe o-(alkyle en C 1-C 4)-phényle, o-halogénophényle ou 2,6-dihalogénophényle dans lesquels les halogènes peuvent tous avoir un numéro atomique allant jusqu'à 35 inclus, alk représente un groupe méthylène, $R_1$ représente l'hydrogène, $R_2$ un groupe carbamoyle et $R_3$ l'hydrogène.

**12.** Le 1-(2,6-difluorobenzyl)-imidazole-4-carboxamide selon revendication 1.

**13.** Le 1-(o-chlorobenzyl)-imidazole-4-carboxamide selon revendication 1.

**14.** Le 1-(2,6-difluorobenzyl)-imidazole-4-(N-méthyl)-carboxamide selon revendication 1.

**15.** Le 1-(o-fluorobenzyl)-imidazole-4-carboxamide selon revendication 1.

**16.** Le 1-(2,6-difluorobenzyl)-imidazole-4-(N,N-diméthyl)-carboxamide selon revendication 1.

**17.** Le 1-(2,6-difluorobenzyl)-imidazole-5-carboxamide selon revendication 1.

**18.** Le 1-(2,6-difluorobenzyl)-imidazole-4,5-dicarboxamide, le 1-(2,6-difluorobenzyl)-imidazole-5-carboxamide, le 1-(2,6-difluorobenzyl)-5-méthyl-imidazole-4-carboxamide, le 1-(2,6-difluorobenzyl)-2-méthyl-imidazole-4-carboxamide, le 1-(o-méthylbenzyl)-imidazole-4-carboxamide, le 1-[1-(2,6-difluorophényl)-éthyl]-imidazole-4-carboxamide ou le 1-(2,6-difluorobenzyl)-imidazole-4-(N-acétyl)-carboxamide selon revendication 1.

**19.** Le 1-(2,6-difluorobenzyl)-imidazole-4-(N-éthyl)-carboxamide ou le 1-(2,6-difluorobenzyl)-imidazole-5-(N-méthyl)-carboxamide selon revendication 1.

**20.** Le 1-(2,6-difluorobenzyl)-imidazole-4,5-di-(N,N-diméthyl)-carboxamide ou le 1-[1-(2,6-difluorophényl)-éthyl]-imidazole-5-carboxamide selon revendication 1.

**21.** Composés de formule

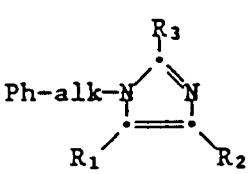

(I),

dans laquelle Ph représente un groupe phényle mono-, di-ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle éventuellement substitué par un ou deux groupes alkyle en C 1-C 7 et l'autre représente l'hydrogène, un groupe alkyle en C 1-C 7 ou un groupe carbamoyle éventuellement substitué par un ou deux groupes alkyle en C 1-C 7, et $R_3$ représente l'hydrogène ou un groupe alkyle en C 1-C 7, sous réserve que, dans les composés de formule I dans lesquels Ph-Alk représente un groupe monohalogénophényl-alkyle en C 1-C 2, $R_3$ représente l'hydrogène lorsque l'un des symboles $R_1$ et $R_2$ représente un groupe carbamoyle et l'autre l'hydrogène, pour l'utilisation dans un procédé pour le traitement thérapeutique de l'organisme humain ou animal.

**22.** Composés selon l'une des revendications 1 à 6 et 11 à 18, pour l'utilisation dans un procédé pour le traitement thérapeutique de l'organisme humain ou animal.

**23.** Composés selon l'une des revendications 8, 9 et 19, pour l'utilisation dans un procédé pour le traitement thérapeutique de l'organisme humain ou animal.

**24.** Composés selon l'une des revendications 7, 10 et 20, pour l'utilisation dans un procédé pour le traitement thérapeutique de l'organisme humain ou animal.

**25.** Compositions pharmaceutiques contenant un composé selon l'une des revendications 1 à 6, 11 à 18, 21 et 22, avec des produits auxiliaires pharmaceutiques usuels.

**26.** Compositions pharmaceutiques contenant un composé selon l'une des revendications 8, 9, 19 et 23, avec des produits auxiliaires pharmaceutiques usuels.

**27.** Compositions pharmaceutiques contenant un composé selon l'une des revendications 7, 10, 20 et 24, avec des produits auxiliaires pharmaceutiques usuels.

**28.** Procédé de préparation des composés de formule

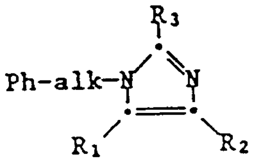

(I),

dans laquelle Ph repr é sente un groupe phényle mono-, di-ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle éventuellement substitué par un ou deux groupes alkyle en C 1-C 7 et l'autre représente l'hydrogène, un groupe alkyle en C 1-C 7 ou un groupe carbamoyle éventuellement substitué par un ou deux groupes alkyle en C 1-C 7, et $R_3$ représente l'hydrogène ou un groupe alkyle en C 1-C 7, sous réserve que dans les composés de formule I dans lesquels $R_1$ et $R_2$ sont identiques et représentent chacun un groupe carbamoyle ou N-méthylcarbamoyle, l'un au moins des substituants de Ph est relié en position ortho lorsque $R_3$ représente l'hydrogène, et sous réserve également que dans les composés de formule I dans lesquels Ph-alk représente un groupe monohalogénophényl-alkyle en C 1-C 2, $R_3$ représente l'hydrogène lorsque l'un des symboles $R_1$ et $R_2$ représente un groupe carbamoyle et l'autre l'hydrogène, caractérisé en ce que :

a) dans un composé de formule

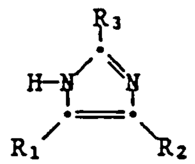

(II)

ou l'un de ses sels, on introduit le groupe de formule Ph-alk- ou bien
b) dans un composé de formule

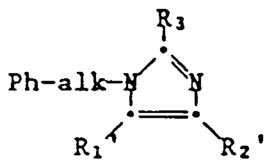

(III),

dans laquelle R'$_1$ représente un groupe $R_1$ ou X et R'$_2$ un groupe X ou R'$_1$ un groupe X et R'$_2$ un groupe $R_2$, X étant un groupe convertible en un groupe carbamoyle éventuellement substitué par un ou deux groupes alkyle en C 1-C 7, on convertit le ou les groupes X en groupes carbamoyle éventuellement substitués par un ou deux groupes alkyle en C 1-C 7, ou bien
c) pour préparer les composés de formule I dans laquelle $R_3$ représente l'hydrogène et $R_2$ un groupe carbamoyle portant deux substituants alkyle en C 1-C 4, on condense un composé de formule

Ph-alk-$NH_2$ (IV)

avec un isonitrile de formule

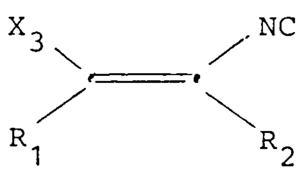

(V),

dans laquelle $X_3$ représente un groupe éliminable nucléofuge, et si on le désire, dans chaque cas, on sépare un mélange d'isomères éventuellement obtenu en les composants et on isole l' isomère recherché de formule I, on convertit un composé de formule I obtenu comme décrit ci-dessus en un autre composé de formule I et/ou on sépare un mélange de diastéréoisomères ou d'énantiomères obtenu comme décrit ci-dessus en les diastéréoisomères ou énantiomères.

**29.** Utilisation d'un composé selon l'une des revendications 1 à 24 pour la préparation de compositions pharmaceutiques servant au traitement de l'épilepsie.

**Revendications pour les Etats contractants suivants : AT, ES, GR**

**1.** Procédé de préparation de composés de formule

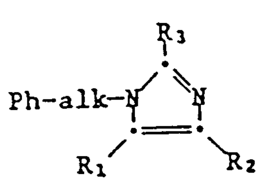

(I),

dans laquelle Ph représente un groupe phényle mono-, di-ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle éventuellement substitué par un ou deux groupes alkyle en C 1-C 7 et l'autre représente l'hydrogène, un groupe alkyle en C 1-C 7 ou un groupe carbamoyle éventuellement substitué par un ou deux groupes alkyle en C 1-C 7, et $R_3$ représente l'hydrogène ou un groupe alkyle en C 1-C 7, sous réserve que dans les composés de formule I dans lesquels $R_1$ et $R_2$ sont identiques et représentent chacun un groupe carbamoyle ou N-méthylcarbamoyle, l'un au moins des substituants de Ph est relié en position ortho lorsque $R_3$ représente l'hydrogène, et sous réserve également que dans les composés de formule I dans lesquels Ph-alk représente un groupe monohalogénophényl-alkyle en C 1-C 2, $R_3$ représente l'hydrogène lorsque l'un des symboles $R_1$ et $R_2$ représente un groupe carbamoyle et l'autre l'hydrogène, caractérisé en ce que :

a) dans un composé de formule

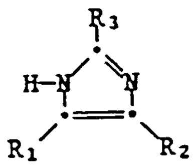

(II)

ou l'un de ses sels, on introduit le groupe de formule Ph-alk- ou bien

b) dans un composé de formule

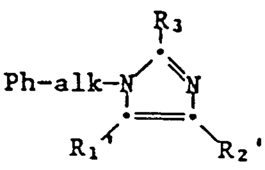

(III),

dans laquelle $R'_1$ représente un groupe $R_1$ ou X et $R'_2$ un groupe X ou $R'_1$ un groupe X et $R'_2$ un groupe $R_2$, X étant un groupe convertible en un groupe carbamoyle éventuellement substitèé par un ou deux groupes alkyle en C 1-C 7, on convertit le ou les groupes X en groupes carbamoyle éventuellement substitués par un ou deux groupes alkyle en C 1-C 7, ou bien

c) pour préparer les composés de formule I dans laquelle $R_3$ représente l'hydrogène et $R_2$ un groupe carbamoyle portant deux substituants alkyle en C 1-C 4, on condense un composé de formule

Ph-alk-$NH_2$ (IV)

avec un isonitrile de formule

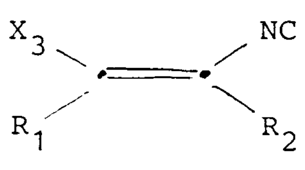

(V),

dans laquelle $X_3$ représente un groupe éliminable nucléofuge, et si on le désire, dans chaque cas, on sépare un mélange d'isomères éventuellement obtenu en les composants et on isole l'isomère recherché de formule I, on convertit un composé de formule I obtenu comme décrit ci-dessus en un autre composé de formule I et/ou on sépare un mélange de diastéréoisomères ou d'énantiomères obtenu comme décrit ci-dessus en les diastéréoisomères ou énantiomères.

**2.** Procédé selon la revendication 1, caractérisé en ce que l'on prépare des composés de formule I dans

laquelle Ph représente un groupe phényle mono-, di- ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle, N-(alkyle en C 1-C 7)-carbamoyle ou N,N-di-(alkyle en C 1-C 4)-carbamoyle et l'autre l'hydrogène, un groupe alkyle en C 1-C 7, carbamoyle, N-(alkyle en C 1-C 7)-carbamoyle ou N,N-di-(alkyle en C 1-C 4)-carbamoyle et $R_3$ représente l'hydrogène ou un groupe alkyle en C 1-C 7.

**3.** Procédé selon la revendication 1, caractérisé en ce que l'on prépare des composés de formule I dans laquelle Ph représente un groupe phényle monosubstitué par un groupe alkyle en C 1-C 4 ou disubstitué par des halogènes de numéro atomique allant jusqu'à 35 inclus ou par un halogène de numéro atomique allant jusqu'à 35 inclus et un groupe alkyle en C 1-C 4, alk représente un groupe alca-1-ylidène en C 1-C 4 ou alca-2-ylidène en C 3-C 4, l'un des symboles $R_1$ et $R_2$ représente un groupe carbamoyle ou N-(alkyle en C 1-C 4)- ou N,N-di-(alkyle en C 1-C 4)-carbamoyle et l'autre l'hydrogène, un groupe carbamoyle ou N-(alkyle en C 1-C 4)- ou N,N-di-(alkyle en C 1-C 4)-carbamoyle et $R_3$ représente l'hydrogène ou un groupe alkyle en C 1-C 4.

**4.** Procédé selon l'une des revendications 1 à 3, caractérisé en ce que l'on prépare des composés de formule I dans lesquels au moins un des substituants mentionnés pour Ph est relié en position ortho.

**5.** Procédé selon la revendication 1, caractérisé en ce que l'on prépare des composés de formule I dans laquelle Ph représente un groupe o-(alkyle en C 1-C 4)-phényle, o-halogénophényle ou 2,6- ou encore 2,5-dihalogénophényle,les halogènes pouvant tous avoir un numéro atomique allant jusqu'à 35 inclus, alk représente un groupe alca-1-ylidène en C 1-C 4, l'un des symboles $R_1$ et $R_2$ représente un groupe carbamoyle, N-(alkyle en C 1-C 4)-carbamoyle ou N,N-di-(alkyle en C 1-C 4)-carbamoyle et l'autre l'hydrogène, un groupe carbamoyle, N-(alkyle en C 1-C 4)-carbamoyle ou N,N-di-(alkyle en C 1-C 4)-carbamoyle et $R_3$ représente l'hydrogène.

**6.** Procédé selon l'une des revendications 1 à 5, caractérisé en ce que l'on prépare des composés de formule I dans laquelle alk représente un groupe méthylène, $R_1$ l'hydrogène ou un groupe carbamoyle et $R_2$ un groupe carbamoyle.

**7.** Procédé selon l'une des revendications 1 à 5, caractérisé en ce que l'on prépare des composés de formule I dans laquelle alk représente un groupe méthylène, $R_1$ l'hydrogène et $R_2$ un groupe carbamoyle.

**8.** Procédé selon la revendication 1, caractérisé en ce que l'on prépare des composés de formule I dans laquelle Ph représente un groupe o-(alkyle en C 1-C 4)-phényle, o-halogénophényle ou 2,6-dihalogénophényle, les halogènes en question pouvant tous avoir un numéro atomique allant jusqu'à 35 inclus, alk représente un groupe méthylène, $R_1$ l'hydrogène, $R_2$ un groupe carbamoyle, N-(alkyle en C 1-C 4)-carbamoyle ou N,N-di-(alkyle en C 1-C 4)-carbamoyle, et $R_3$ l'hydrogène.

**9.** Procédé selon la revendication 1, caractérisé en ce que l'on prépare des composés de formule I dans laquelle Ph représente un groupe 2,6-dihalogénophényle dont les halogènes peuvent tous deux avoir un numéro atomique allant jusqu'à 35 inclus, alk représente un groupe méthylène, $R_1$ un groupe carbamoyle, N-(alkyle en C 1-C 4)-carbamoyle ou N,N-di-(alkyle en C 1-C 4)-carbamoyle, $R_2$ représente l'hydrogène, un groupe alkyle en C 1-C 4, carbamoyle ou bien, comme $R_1$, un groupe N-(alkyle en C 1-C 4)-carbamoyle ou N,N-di-(alkyle en C 1-C 4)-carbamoyle et $R_3$ représente l'hydrogène

**10.** Procédé selon la revendication 1, caractérisé en ce que l'on prépare des composés de formule I dans laquelle Ph représente un groupe 2,6-dihalogénophényle dont les halogènes peuvent tous deux avoir un numéro atomique allant jusqu'à 35 inclus, alk représente un groupe méthylène et $R_3$ l'hydrogène, et dans lesquels ou bien $R_1$ représentel'hydrogène et $R_2$ un groupe carbamoyle, N-(alkyle en C 1-C 4)-carbamoyle ou N,N-di-(alkyle en C 1-C 4)-carbamoyle, ou bien $R_1$ représente un groupe carbamoyle ou N-(alkyle en C 1-C 4)-carbamoyle et $R_2$ l'hydrogène, ou bien $R_1$ et $R_2$ représentent tous deux des groupes carbamoyle.

**11.** Procédé selon la revendication 1, caractérisé en ce que l'on prépare des composés de formule I dans laquelle Ph représente un groupe o-(alkyle en C 1-C 4)-phényle, o-halogénophényle ou 2,6-dihalogénophényle dans lesquels les halogènes peuvent tous avoir un numéro atomique allant jusqu'à 35 inclus, alk représente un groupe méthylène, $R_1$ représente l'hydrogène, $R_2$ un groupe carbamoyle et $R_3$ l'hydrogène.

**12.** Procédé selon la revendication 1, pour la préparation du 1-(2,6-difluorobenzyl)-imidazole-4-carboxamide.

**13.** Procédé selon la revendication 1, pour la préparation du 1-(o-chlorobenzyl)-imidazole-4-carboxamide.

**14.** Procédé selon la revendication 1, pour la préparation du 1-(2,6-difluorobenzy1)-imidazole-4-(N-méthyl)-carboxamide.

**15.** Procédé selon la revendication 1, pour la préparation du 1-(o-fluorobenzyl)-imidazole-4carboxamide.

**16.** Procédé selon la revendication 1, pour la préparation du 1-(2,6-difluorobenzyl)-imidazole-4-(N,N-diméthyl)-carboxamide.

**17.** Procédé selon la revendication 1, pour la préparation du 1-(2,6-difluorobenzyl)-imidazole-5-carboxamide.

**18.** Procédé selon la revendication 1, pour la préparation du 1-(2,6-difluorobenzyl)-imidazole-4,5-dicarboxamide, du 1-(2,6-difluorobenzyl)-imidazole-5-carboxamide, du 1-(2,6-difluorobenzyl)-5-méthyl-imidazole-4-carboxamide, du 1-(2,6-difluorobenzyl)-2-méthyl-imidazole-4-carboxamide, du 1-(o-méthylbenzyl)-imidazole-4-carboxamide, du 1-[1-(2,6-difluorophényl)-éthyl]-imidazole-4-carboxamide, du 1-(2,6-difluorobenzyl)-imidazole-4-(N-acétyl)-carboxamide, du 1-(2,6-difluorobenzyl)-imidazole-4-(N-éthyl)-carboxamide, du 1-(2,6-difluorobenzyl)-imidazole-5-(N-méthyl)-carboxamide, du 1-(2,6-difluorobenzyl)-imidazole-4,5-di-(N,N-diméthyl)-carboxamide, du 1-[1-(2,6-difluorophényl)-éthyl]-imidazole-5-carboxamide.

**19.** Procédé de préparation de compositions pharmaceutiques, caractérisé en ce que l'on mélange avec des produits auxiliaires pharmaceutiques usuels un composé de formule

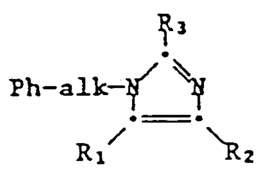

(I),

dans laquelle Ph représente un groupe phényle mono-, di-ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle éventuellement substitué par un ou deux groupes alkyle en C 1-C 7 et l'autre l'hydrogène, un groupe alkyle en C 1-C 7 ou un groupe carbamoyle éventuellement substitué par un ou deux groupes alkyle en C 1-C 7, et $R_3$ représente l'hydrogène ou un groupe alkyle en C 1-C 7, sous réserve que dans les composés de formule I dans laquelle Ph-alk représente un groupe monohalogénophényl-alkyle en C 1-C 2, $R_3$ représente l'hydrogène lorsque l'un des symboles $R_1$ et $R_2$ représente un groupe carbamoyle et l'autre l'hydrogène.

**20.** Procédé de préparation de compositions pharmaceutiques, caractérisé en ce que :

a) dans un composé de formule

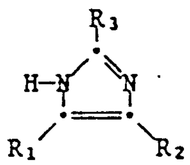

(II)

ou l'un de ses sels, on introduit le groupe de formule Ph-alk- ou bien

b) dans un composé de formule

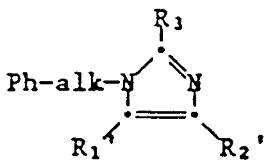

(III),

dans laquelle $R'_1$ représente un groupe $R_1$ ou X et $R'_2$ un groupe X ou $R'_1$ un groupe X et $R'_2$ un groupe $R_2$, X étant un groupe convertible en un groupe carbamoyle éventuellement substitué par un ou deux groupes alkyle en C 1-C 7, on convertit le ou les groupes X en groupes carbamoyle éventuellement substitués par un ou deux groupes alkyle en C 1-C 7, ou bien

c) pour préparer les composés de formule I dans laquelle $R_3$ représente l'hydrogène et $R_2$ un groupe carbamoyle portant deux substituants alkyle en C 1-C 4, on condense un composé de formule

$Ph\text{-}Alk\text{-}NH_2$ (IV)

avec un isonitrile de formule

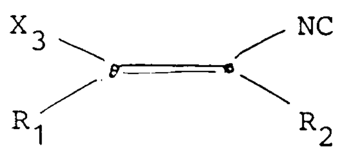

(V),

dans laquelle $X_3$ représente un groupe éliminable nucléofuge, et si on le désire, dans chaque cas, on sépare un mélange d'isomères éventuellement obtenu en les composants et on isole l'isomère recherché de formule I, on convertit un composé de formule I obtenu comme décrit ci-dessus en un autre composé de formule I et/ou on sépare un mélange de diastéréoisomères ou d'énantiomères obtenu comme décrit ci-dessus en les diastéréoisomères ou énantiomères,

et on mélange le composé obtenu, répondant à la formule

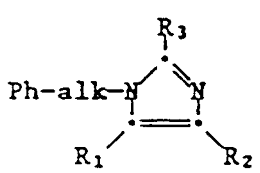

(I),

dans laquelle Ph représente un groupe phényle mono-, di-ou tri-substitué par des groupes alkyle en C 1-C 7 et/ou des halogènes de numéro atomique allant jusqu'à 35 inclus, alk représente un groupe alkylidène en C 1-C 4, l'un des symboles $R_1$ et $R_2$ représente un groupe carbamoyle éventuellement substitué par un ou deux groupes alkyle en C 1-C 7 et l'autre représente l'hydrogène, un groupe alkyle en C 1-C 7 ou un groupe carbamoyle éventuellement substitué par un ou deux groupes alkyle en C 1-C 7, et $R_3$ représente l'hydrogène ou un groupe alkyle en C 1-C 7, sous

réserve que dans les composés de formule I dans laquelle Ph-alk représente un groupe monohalogénophénylalkyle en C 1-C 2, $R_3$ représente l'hydrogène lorsque l'un des symboles $R_1$ et $R_2$ représente un groupe carbamoyle et l'autre l'hydrogène,
avec des produits auxiliaires pharmaceutiques usuels.